# NEW EUROPEAN PATENT SPECIFICATION

(11) **EP 0 781 329 B2**
(45) Date of publication and mention of the opposition decision: **29.12.2010**
(45) Mention of the grant of the patent: 02.08.2006
(21) Application number: 95931798.3
(22) Date of filing: 08.09.1995
(51) Int. Cl.: C12N 15/09, C12N 15/12, C12N 15/33, C12N 15/64

(54) **OVEREXPRESSION OF MAMMALIAN AND VIRAL PROTEINS**
ÜBEREXPRESSION VON SÄUGETIER- UND VIRUSPROTEINEN
SUREXPRESSION DE PROTEINES MAMMALIENNES ET VIRALES

(30) Priority: 19.09.1994 US 324243
(43) Date of publication of application: 02.07.1997
(73) Proprietor: THE GENERAL HOSPITAL CORPORATION, Boston, MA 02114 (US)
(72) Inventor: SEED, Brian, Boston, MA 02114 (US)
(74) Representative: Lee, Nicholas John
(86) International application number: PCT/US1995/011511
(87) International publication number: WO 1996/009378

(56) References cited:
- EP-A- 0 345 242
- WO-A-97/11086
- WO-A-98/12207
- US-A- 5 270 171
- HOLLER T P ET AL.: "HIV1 integrase expressed in Escherichia coli from a synthetic gene" GENE., vol. 136, 1993, pages 323-328, XP002078376 AMSTERDAM NL
- SCORER C A ET AL: "The intracellular production and secretion of HIV-1 envelope protein in the methylotrophic yeast Pichia pastoris" GENE., vol. 136, 1993, pages 111-119, XP002078377 AMSTERDAM NL
- NUCLEIC ACIDS RESEARCH, Volume 18, Number 4, issued 1990, McCARRY, "Molecular Evolution of the Human PgK-2 Retroposon", pages 949-955.
- JAPANESE JOURNAL OF CANCER RESEARCH, Volume 80, issued March 1989, KAMIYA et al., "Transformation of NIH3T3 Cells with Synthetic C-Ha-Ras Genes", pages 200-203.
- NUCLEIC ACIDS RESEARCH, Volume 16, Number 17, issued 1988, SHARP et al., "Codon Usage Patterns in Escherichia Coli, Bacillus Subtilis, Saccharomyces Cerevisiae, Schizosaccharomyces Pombe, Drosophila Melanogaster and Homo Sapiens: a Review of the Considerable within-Species Diversity", pages 8207-8211.
- PROC. NATL. ACAD. SCI. U.S.A., Volume 83, issued November 1986, NEWGARD et al., "Sequence Analysis of the cDNA Encoding Human Liver Glycogen Phosphorylase Reveals Tissue-Specific Codon Usage", pages 8132-8136.
- GENE, Volume 46, issued 1986, COULOMBE et al., "Expression of a Synthetic Human Interferon-alpha1 Gene with Modified Nucleotide Sequence in Mammalian Cells", pages 89-95.
- EDGE ET AL: "Total synthesis of human leukocyte interferpn gene." NATURE, vol. 292, , -

### SEQUENCE LISTING

(1) GENERAL INFORMATION:
   (i) APPLICANT: SEED, BRIAN
   (ii) TITLE OF INVENTION: OVEREXPRESSION OF MAMMALIAN AND VIRAL PROTEINS
   (iii) NUMBER OF SEQUENCES: 37
   (iv) CORRESPONDENCE ADDRESS:
      (A) ADDRESSEE: Fish & Richardson
      (B) STREET: 225 Franklin Street
      (C) CITY: Boston
      (D) STATE: Massachusetts
      (E) COUNTRY: U.S.A.
      (F) ZIP: 02110-2804
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30B
   (vi) CURRENT APPLICATION DATA:
      (A) APPLICATION NUMBER: 08/308,286
      (B) FILING DATE: 19-SEP-1994
   (viii) ATTORNEY/AGENT INFORMATION:
      (A) NAME: CLARK, PAUL T
      (B) REGISTRATION NUMBER: 30,162
      (C) REFERENCE/DOCKET NUMBER: 00786/226001
   (ix) TELECOMMUNICATION INFORMATION:
      (A) TELEPHONE: (617) 542-5070
      (B) TELEFAX: (617) 542-8906
      (C) TELEX: 200154
(2) INFORMATION FOR SEQ ID NO:1:
   (i) SEQUENCE CHARACTERISTICS:
      (A) LENGTH: 24 base pairs
      (B) TYPE: nucleic acid
      (C) STRANDEDNESS: single
      (D) TOPOLOGY: linear
   (xi) SEQUENCE DESCRIPTION: SEQ ID NO:1:
      CGCGGGCTAG CCACCGAGAA GCTG 24
(2) INFORMATION FOR SEQ ID NO:2:
   (i) SEQUENCE CHARACTERISTICS:
      (A) LENGTH: 196 base pairs
      (B) TYPE: nucleic acid
      (C) STRANDEDNESS: single
      (D) TOPOLOGY: linear
   (xi) SEQUENCE DESCRIPTION: SEQ ID NO:2:
   ACCGAGAAGC TGTGGGTGAC CGTGTACTAC GGCGTGCCCG TGTGGAAGAG AGGCCACCAC 60
   CACCCTGTTC TGCGCCAGCG ACGCCAAGGC GTACGACACC GAGGTGCACA ACGTGTGGGC 120
   CACCCAGGCG TGCGTGCCCA CCGACCCCAA CCCCCAGGAG GTGGAGCTCG TGAACGTGAC 180
   CGAGAACTTC AACATG 196
(2) INFORMATION FOR SEQ ID NO:3:
   (i) SEQUENCE CHARACTERISTICS:
      (A) LENGTH: 34 base pairs
      (B) TYPE: nucleic acid
      (C) STRANDEDNESS: single (D),TOPOLOGY: linear
   (xi) SEQUENCE DESCRIPTION: SEQ ID NO:3:
      CCACCATGTT GTTCTTCCAC ATGTTGAAGT TCTC 34
(2) INFORMATION FOR SEQ ID NO:4:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:4:
      GACCGAGAAC TTCAACATGT GGAAGAACAA CAT 33
(2) INFORMATION FOR SEQ ID NO:5:
   (i) SEQUENCE CHARACTERISTICS:
      (A) LENGTH: 192 base pairs
      (B) TYPE: nucleic acid
      (C) STRANDEDNESS: single
      (D) TOPOLOGY: linear
   (xi) SEQUENCE DESCRIPTION: SEQ ID NO:5:
   TGGAAGAACA ACATGGTGGA GCAGATGCAT GAGGACATCA TCAGCCTGTG GGACCAGAGC 60
   CTGAAGCCCT GCGTGAAGCT GACCCCCTGT GCGTGACCTG AACTGCACCG ACCTGAGGAA 120
   CACCACCAAC ACCAACACAG CACCGCCAAC AACAACAGCA ACAGCGAGGG CACCATCAAG 180
   GGCGGCGAGA TG 192
(2) INFORMATION FOR SEQ ID NO:6:
   (i) **SEQUENCE CHARACTERISTICS:**
      (A) LENGTH: 33 base pairs
      (B) TYPE: nucleic acid
      (C) STRANDEDNESS: single
      (D) TOPOLOGY: linear
   (xi) SEQUENCE DESCRIPTION: SEQ ID NO:6:
      GTTGAAGCTG CAGTTCTTCA TCTCGCCGCC CTT 33
(2) INFORMATION FOR SEQ ID NO:7:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (xi) SEQUENCE DESCRIPTION: SEQ ID NO:7:
      GAAGAACTGC AGCTTCAACA TCACCACCAG C 31
(2) INFORMATION FOR SEQ ID NO:8:
   (i) SEQUENCE CHARACTERISTICS:
      (A) LENGTH: 195 base pairs
      (B) TYPE: nucleic acid
      (C) STRANDEDNESS: single
      (D) TOPOLOGY: linear
   (xi) SEQUENCE DESCRIPTION: SEQ ID NO:8:
   AACATCACCA CCAGCATCCG CGACAAGATG CAGAAGGAGT ACGCCCTGCT GTACAAGCTG 60
   GATATCGTGA GCATCGACAA CGACAGCACC AGCTACCGCC TGATCTCCTG CAACACCAGC 120
   GTGATCACCC AGGCCTGCCC CAAGATCAGC TTCGAGCCCA TCCCCATCCA CTACTGCGCC 180
   CCCGCCGGCT TCGCC 195
(2) INFORMATION FOR SEQ ID NO:9:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:9:
      GAACTTCTTG TCGGCGGCGA AGCCGGCGGG 30
(2) INFORMATION FOR SEQ ID NO:10:
   (i) SEQUENCE CHARACTERISTICS:
      (A) LENGTH: 47 base pairs
      (B) TYPE: nucleic acid
      (C) STRANDEDNESS: single
      (D) TOPOLOGY: linear
   (xi) SEQUENCE DESCRIPTION: SEQ ID NO:10:
      GCGCCCCCGC CGGCTTCGCC ATCCTGAAGT GCAACGACAA GAAGTTC 47
(2) INFORMATION FOR SEQ ID NO:11:
   (i) SEQUENCE CHARACTERISTICS:
      (A) LENGTH: 198 base pairs
      (B) TYPE: nucleic acid
      (C) STRANDEDNESS: single
      (D) TOPOLOGY: linear
   (xi) SEQUENCE DESCRIPTION: SEQ ID NO:11:
   GCCGACAAGA AGTTCAGCGG CAAGGGCAGC TGCAAGAACG TGAGCACCGT GCAGTGCACC 60
   CACGGCATCC GGCCGGTGGT GAGCACCCAG CTCCTGCTGA ACGGCAGCCT GGCCGAGGAG 120
   GAGGTGGTGA TCCGCAGCGA GAACTTCACC GACAACGCCA AGACCATCAT CGTGCACCTG 180
   AATGAGAGCG TGCAGATC 198
(2) INFORMATION FOR SEQ ID NO:12:
   (i) SEQUENCE CHARACTERISTICS:
      (A) LENGTH: 34 base pairs a
      (B) TYPE: nucleic acid
      (C) STRANDEDNESS: single
      (D) TOPOLOGY: linear
   (xi) SEQUENCE DESCRIPTION: SEQ ID NO:12:
      AGTTGGGACG CGTGCAGTTG ATCTGCACGC TCTC 34
(2) INFORMATION FOR SEQ ID NO:13:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:13:
      GAGAGCGTGC AGATCAACTG CACGCGTCCC 30
(2) INFORMATION FOR SEQ ID NO:14:
   (i) SEQUENCE CHARACTERISTICS:
      (A) LENGTH: 120 base pairs
      (B) TYPE: nucleic acid
      (C) STRANDEDNESS: single
      (D) TOPOLOGY: linear
   (xi) SEQUENCE DESCRIPTION: SEQ ID NO:14:
   AACTGCACGC GTCCCAACTA CAACAAGCGC AAGCGCATCC ACATCGGCCC CGGGCGCGCC 60
   TTCTACACCA CCAAGAACAT CATCGGCACC ATCCTCCAGG CCCACTGCAA CATCTCTAGA 120
(2) INFORMATION FOR SEQ ID NO:15:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:15:
      GTCGTTCCAC TTGGCTCTAG AGATGTTGCA 30
(2) INFORMATION FOR SEQ ID NO:16:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (xi) SEQUENCE DESCRIPTION: SEQ ID NO:16:
      GCAACATCTC TAGAGCCAAG TGGAACGAC 29
(2) INFORMATION FOR SEQ ID NO:17:
   (i) SEQUENCE CHARACTERISTICS:
      (A) LENGTH: 131 base pairs
      (B) TYPE: nucleic acid
      (C) STRANDEDNESS: single
      (D) TOPOLOGY: linear
   (xi) SEQUENCE DESCRIPTION: SEQ ID NO:17:
   GCCAAGTGGA ACGACACCCT GCGCCAGATC GTGAGCAAGC TGAAGGAGCA GTTCAAGAAC 60
   AAGACCATCG TGTTCACCAG AGCAGCGGCG GCGACCCCGA GATCGTGATG CACAGCTTCA 120
   ACTGCGGCGG C 131
(2) INFORMATION FOR SEQ ID NO:18:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (xi) SEQUENCE DESCRIPTION: SEQ ID NO:18:
      GCAGTAGAAG AATTCGCCGC CGCAGTTGA 29
(2) INFORMATION FOR SEQ ID NO:19:
   (i) SEQUENCE CHARACTERISTICS:
      (A) LENGTH: 29 base pairs
      (B) TYPE: nucleic acid
      (C) STRANDEDNESS: single
      (D) TOPOLOGY: linear
   (xi) SEQUENCE DESCRIPTION: SEQ ID NO:19:
      TCAACTCCGG CGGCGAATTC TTCTACTGC 29
(2) INFORMATION FOR SEQ ID NO:20:
   (i) SEQUENCE CHARACTERISTICS:
      (A) LENGTH: 195 base pairs
      (B) TYPE: nucleic acid
      (C) STRANDEDNESS: single
      (D) TOPOLOGY: linear
   (xi) SEQUENCE DESCRIPTION: SEQ ID NO:20:
   GGCGAATTCT TCTACTGCAA CACCAGCCCC CTGTTCAACA GCACCTGGAA CGGCAACAAC 60
   ACCTGGAACA ACACCACCGG CAGCAACAAC AATATTACCC TCCAGTGCAA GATCAAGCAG 120
   ATCATCAACA TGTGGCAGGA GGTGGGCAAG GCCATGTACG CCCCCCCCAT CGAGGGCCAG 180
   ATCCGGTGCA GCAGC 195
(2) INFORMATION FOR SEQ ID NO:21:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (xi) SEQUENCE DESCRIPTION: SEQ ID NO:21:
      GCAGACCGGT GATGTTGCTG CTGCACCGGA TCTGGCCCTC 40
(2) INFORMATION FOR SEQ ID NO:22:
   (i) SEQUENCE CHARACTERISTICS:
      (A) LENGTH: 40 base pairs
      (B) TYPE: nucleic acid
      (C) STRANDEDNESS: single
      (D) TOPOLOGY: linear
   (xi) SEQUENCE DESCRIPTION: SEQ ID NO:22:
      CGAGGGCCAG ATCCGGTGCA GCAGCAACAT CACCGGTCTG 40
(2) INFORMATION FOR SEQ ID NO:23:
   (i) SEQUENCE CHARACTERISTICS:
      (A) LENGTH: 242 base pairs
      (B) TYPE: nucleic acid
      (C) STRANDEDNESS: single
      (D) TOPOLOGY: linear
   (xi) SEQUENCE DESCRIPTION: SEQ ID NO:23:
   AACATCACCG GTCTGCTGCT GCTGCTGACC CGGACGGCGG CAAGGACACC GACACCAACG 60
   ACACCGAAAT CTTCCGCGAC GGCGGCAAGG ACACCAACGA CACCGAAATC TTCCGCCCCG 120
   GCGGCGGCGA CATGCGCGAC AACTGGAGAT CTGAGCTGTA CAAGTACAAG GTGGTGACGA 180
   TCGAGCCCCT GGGCGTGGCC CCCACCAAGG CCAAGCGCGC GGTGGTGCAG CGCGAGAAGC 240
   GC 242
(2) INFORMATION FOR SEQ ID NO:24:
   (i) SEQUENCE CHARACTERISTICS:
      (A) LENGTH: 38 base pairs
      (B) TYPE: nucleic acid
      (C) STRANDEDNESS: single
      (D) TOPOLOGY: linear
   (xi) SEQUENCE DESCRIPTION: SEQ ID NO:24:
      CGCGGGCGGC CGCTTTAGCG CTTCTCGCGC TGCACCAC 38
(2) INFORMATION FOR SEQ ID NO:25:
   (i) SEQUENCE CHARACTERISTICS:
      (A) LENGTH: 39 base pairs
      (B) TYPE: nucleic acid
      (C) STRANDEDNESS: single
      (D) TOPOLOGY: linear
   (xi) SEQUENCE DESCRIPTION: SEQ ID NO:25:
      CGCGGGGGAT CCAAGCTTAC CATGATTCCA GTAATAAGT 39
(2) INFORMATION FOR SEQ ID NO:26:
   (i) SEQUENCE CHARACTERISTICS:
      (A) LENGTH: 165 base pairs
      (B) TYPE: nucleic acid
      (C) STRANDEDNESS: single
      (D) TOPOLOGY: linear
   (xi) SEQUENCE DESCRIPTION: SEQ ID NO:26:
   ATGAATCCAG TAATAAGTAT AACATTATTA TTAAGTGTAT TACAAATGAG TAGAGGACAA 60
   AGAGTAATAA GTTTAACAGC ATCTTTAGTA AATCAAAATT TGAGATTAGA TTGTAGACAT 120
   GAAAATAATA CAAATTTGCC AATACAACAT GAATTTTCAT TAACG 165
(2) INFORMATION FOR SEQ ID NO:27:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO:27:
      CGCGGGGAAT TCACGCGTTA ATGAAAATTC ATGTTG 36
(2) INFORMATION FOR SEQ ID NO:28:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:28:
      CGCGGATCCA CGCGTGAAAA AAAAAAACAT 30
(2) INFORMATION FOR SEQ ID NO:29:
   (i) SEQUENCE CHARACTERISTICS:
      (A) LENGTH: 149 base pairs
      (B) TYPE: nucleic acid
      (C) STRANDEDNESS: single
      (D) TOPOLOGY: linear
   (xi) SEQUENCE DESCRIPTION: SEQ ID NO:29:
   CGTGAAAAAA AAAAACATGT ATTAAGTGGA ACATTAGGAG TACCAGAACA TACATATAGA 60
   AGTAGAGTAA TTTGTTTAGT GATAGATTCA TAAAAGTATT AACATTAGCA AATTTTACAA 120
   CAAAAGATGA AGGAGATTAT ATGTGTGAG 149
(2) INFORMATION FOR SEQ ID NO:30:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:30:
      CGCGAATTCG AGCTCACACA TATAATCTCC 30
(2) INFORMATION FOR SEQ ID NO:31:
   (i) SEQUENCE CHARACTERISTICS:
      (A) LENGTH: 30 base pairs
      (B) TYPE: nucleic acid
      (C) STRANDEDNESS: single
      (D) TOPOLOGY: linear
   (xi) SEQUENCE DESCRIPTION: SEQ ID NO:31:
      CGCGGATCCG AGCTCAGAGT AAGTGGACAA 30
(2) INFORMATION FOR SEQ ID NO:32:
   (i) SEQUENCE CHARACTERISTICS:
      (A) LENGTH: 170 base pairs
      (B) TYPE: nucleic acid
      (C) STRANDEDNESS: single
      (D) TOPOLOGY: linear
   (xi) SEQUENCE DESCRIPTION: SEQ ID NO:32:
   CTCAGAGTAA GTGGACAAAA TCCAACAAGT AGTAATAAAA CAATAAATGT AATAAGAGAT 60
   AAATTAGTAA AATGTGAGGA ATAAGTTTAT TAGTACAAAA TACAAGTTGG TTATTATTAT 120
   TATTATTAAG TTTAAGTTTT TTACAAGCAA CAGATTTTAT AAGTTTATGA 170
(2) INFORMATION FOR SEQ ID NO:33:
   (i) SEQUENCE CHARACTERISTICS:
      (A) LENGTH: 36 base pairs
      (B) TYPE: nucleic acid
      (C) STRANDEDNESS: single
      (D) TOPOLOGY: linear
   (xi) SEQUENCE DESCRIPTION: SEQ ID NO:33:
      CGCGAATTCG CGGCCGCTTC ATAAACTTAT AAAATC 36
(2) INFORMATION FOR SEQ ID NO:34:
   (i) SEQUENCE CHARACTERISTICS:
      (A) LENGTH: 1632 base pairs
      (B) TYPE: nucleic acid
      (C) STRANDEDNESS: single
      (D) TOPOLOGY: linear
   (xi) SEQUENCE DESCRIPTION: SEQ ID NO:34:
   CTCGAGATCC ATTGTGCTCT AAAGGAGATA CCCGGCCAGA CACCCTCACC TGCGGTGCCC 60
   AGCTGCCCAG GCTGAGGCAA GAGAAGGCCA GAAACCATGC CCATGGGGTC TCTGCAACCG 120
   CTGGCCACCT TGTACCTGCT GGGGATGCTG GTCGCTTCCG TGCTAGCCAC CGAGAAGCTG 180
   TGGGTGACCG TGTACTACGG CGTGCCCGTG TGGAAGGAGG CCACCACCAC CCTGTTCTGC 240
   GCCAGCGACG CCAAGGCGTA CGACACCGAG GTGCACAACG TGTGGGCCAC CCAGGCGTGC 300
   GTGCCCACCG ACCCCAACCC CCAGGAGGTG GAGCTCGTGA ACGTGACCGA GAACTTCAAC 360
   ATGTGGAAGA ACAACATGGT GGAGCAGATG CATGAGGACA TCATCAGCCT GTGGGACCAG 420
   AGCCTGAAGC CCTGCGTGAA GCTGACCCCC CTGTGCGTGA CCCTGAACTG CACCGACCTG 480
   AGGAACACCA CCAACACCAA CAACAGCACC GCCAACAACA ACAGCAACAG CGAGGGCACC 540
   ATCAAGGGCG GCGAGATGAA CAACTGCAGC TTCAACATCA CCACCAGCAT CCGCGACAAG 600
   ATGCAGAAGG AGTACGCCCT GCTGTACAAG CTGGATATCG TGAGCATCGA CAACGACAGC 660
   ACCAGCTACC GCCTGATCTC CTGCAACACC AGCGTGATCA CCCAGGCCTG GCCCAAGATC 720
   AGCTTCGAGC CCATCCCCAT CCACTACTGC GCCCCCGCCG GCTTCGCCAT CCTGAAGTGC 780
   AACGACAAGA AGTTCAGCGG CAAGGGCAGC TGCAAGAACG TGAGCACCGT GCAGTGCACC 840
   CACGGCATCC GGCCGGTGGT GAGCACCCAG CTCCTGCTGA ACGGCAGCCT GGCCGAGGAG 900
   GAGGTGGTGA TCCGCAGCGA GAACTTCACC GACAACGCCA AGACCATCAT CGTGCACCTG 960
   AATGAGAGCG TGCAGATCAA CTGCACGCGT CCCAACTACA ACAAGCGCAA GCGCATCCAC 1020
   ATCGGCCCCG GGCGCGCCTT CTACACCACC AAGAACATCA TCGGCACCAT CCGCCAGGCC 1080
   CACTGCAACA TCTCTAGAGC CAAGTGGAAC GACACCCTGC GCCAGATCGT GAGCAAGCTG 1140
   AAGGAGCAGT TCAAGAACAA GACCATCGTG TTCAACCAGA GCAGCGGCGG CGACCCCGAG 1200
   ATCGTGATGC ACAGCTTCAA CTGCGGCGGC GAATTCTTCT ACTGCAACAC CAGCCCCCTG 1260
   TTCAACAGCA CCTGGAACGG CAACAACACC TGGAACAACA CCACCGGCAG CAACAACAAT 1320
   ATTACCCTCC AGTGCAAGAT CAAGCAGATC ATCAACATGT GGCAGGAGGT GGGCAAGGCC 1380
   ATGTACGCCC CCCCCATCGA GGGCCAGATC CGGTGCAGCA GCAACATCAC CGGTCTGCTG 1440
   CTGACCCGCG ACGGCGGCAA GGACACCGAC ACCAACGACA CCGAAATCTT CCGCCCCGGC 1500
   GGCGGCGACA TGCGCGACAA CTGGAGATCT GAGCTGTACA AGTACAAGGT GGTGACGATC 1560
   GAGCCCCTGG GCGTGGCCCC CACCAAGGCC AAGCGCCGCG TGGTGCAGCG CGAGAAGCGC 1620
   TAAAGCGGCC GC 1632
(2) INFORMATION FOR SEQ ID NO:35:
   (i) SEQUENCE CHARACTERISTICS:
      (A) LENGTH: 2481 base pairs
      (B) TYPE: nucleic acid
      (C) STRANDEDNESS: single
      (D) TOPOLOGY: linear
   (xi) SEQUENCE DESCRIPTION: SEQ ID NO:35:
   ACCGAGAAGC TGTGGGTGAC CGTGTACTAC GGCGTGCCCG TGTGGAAGGA GGCCACCACC    60
   ACCCTGTTCT GCGCCAGCGA CGCCAAGGCG TACGACACCG AGGTGCACAA CGTGTGGGCC   120
   ACCCAGGCGT GCGTGCCCAC CGACCCCAAC CCCCAGGAGG TGGAGCTCGT GAACGTGACC   180
   GAGAACTTCA ACATGTGGAA GAACAACATG CTGGAGCAGA TGCATGAGGA CATCATCAGC   240
   CTGTGGGACC AGAGCCTGAA GCCCTGCGTG AAGCTGACCC CCCTGTGCGT GACCCTGAAC   300
   TGCACCGACC TGAGGAACAC CACCAACACC AACAACAGCA CCGCCAACAA CAACAGCAAC   360
   AGCGAGGGCA CCATCAAGGG CGGCGAGATG AAGAACTGCA GCTTCAACAT CACCACCAGC   420
   ATCCGCGACA AGATGCAGAA GGAGTACGCC CTGCTGTACA AGCTGGATAT CGTGAGCATC   480
   CACAACGACA GCACCAGCTA CCGCCTGATC TCCTGCAACA CCAGCGTGAT CACCCAGGCC   540
   TGCCCCAAGA TCAGCTTCGA GCCCATCCCC ATCCACTACT GCGCCCCCGC CGGCTTCGCC   600
   ATCCTGAAGT GCAACGACAA GAAGTTCAGC GGCAAGGGCA GCTGCAAGAA CGTGACCACC   660
   GTGCAGTGCA CCCACGGCAT CCGGCCGGTG GTGAGCACCC AGCTCCTGCT GAACGGCAGC 720
   CTGGCCGAGG AGGAGGTGGT GATCCGCAGC GAGAACTTCA CCGACAACGC CAAGACCATC 780
   ATCGTGCACC TGAATGAGAG CGTGCAGATC AACTGCACGC GTCCCAACTA CAACAAGCGC 840
   AAGCGCATCC ACATCGGCCC CGGGCGCGCC TTCTACACCA CCAAGAACAT CATCGGCACC 900
   ATCCGCCAGG CCCACTGCAA CATCTCTAGA GCCAAGTGGA ACGACACCCT GCGCCAGATC 960
   GTGAGCAAGC TGAAGGAGCA GTTCAAGAAC AAGACCATCG TGTTCAACCA GAGCAGCGGC 1020
   GGCGACCCCG AGATCGTGAT GCACAGCTTC AACTGCGGCG GCGAATTCTT CTACTGCAAC 1080
   ACCAGCCCCC TGTTCAACAG CACCTGGAAC GGCAACAACA CCTGGAACAA CACCACCGGC 1140
   AGCAACAACA ATATTACCCT CCAGTGCAAG ATCAAGCAGA TCATCAACAT GTGGCAGGAG 1200
   GTGGGCAAGG CCATGTACGC CCCCCCCATC GAGGGCCAGA TCCGGTGCAG CAGCAACATC 1260
   ACCGGTCTGC TGCTGACCCG CGACGGCGGC AAGGACACCG ACACCAACGA CACCGAAATC 1320
   TTCCGCCCCG GCGGCGGCGA CATGCGCGAC AACTGGAGAT CTGAGCTGTA CAAGTACAAG 1380
   GTGGTGACGA TCGAGCCCCT GGGCGTGGCC CCCACCAAGG CCAAGCGCCG CGTGGTGCAG 1440
   CGCGAGAAGC GGGCCGCCAT CGGCGCCCTG TTCCTGGGCT TCCTGGGGGC GGCGGGCAGC 1500
   ACCATGGGGG CCGCCAGCGT GACCCTGACC GTGCAGGCCC GCCTGCTCCT GAGCGGCATC 1560
   GTGCAGCAGC AGAACAACCT CCTCCGCGCC ATCGAGGCCC AGCAGCATAT GCTCCAGCTC 1620
   ACCGTGTGGG GCATCAAGCA GCTCCAGGCC CGCGTGCTGG CCGTGGAGCG CTACCTGAAG 1680
   GACCAGCAGC TCCTGGGCTT CTGGGGCTGC TCCGGCAAGC TGATCTGCAC CACCACGGTA 1740
   CCCTGGAACG CCTCCTGGAG CAACAAGAGC CTGGACGACA TCTGGAACAA CATGACCTGG 1800
   ATGCAGTGGG AGCGCGAGAT CGATAACTAC ACCAGCCTGA TCTACAGCCT GCTGGAGAAG 1860
   AGCCAGACCC AGCAGGAGAA GAACGAGCAG GAGCTGCTGG AGCTGGACAA CTGGGCGAGC 1920
   CTGTGGAACT GGTTCGACAT CACCAACTGG CTGTGGTACA TCAAAATCTT CATCATGATT 1980
   GTGGGCGGCC TGGTGGGCCT CCGCATCGTG TTCGCCGTGC TGAGCATCGT GAACCGCGTG 2040
   CGCCAGGGCT ACAGCCCCCT GAGCCTCCAG ACCCGGCCCC CCGTGCCGCG CGGGCCCGAC 2100
   CGCCCCGAGG GCATCGAGGA GGAGGGCGGC GAGCGCGACC GCGACACCAG CGGCAGGCTC 2160
   GTGCACGGCT TCCTGGCGAT CATCTGGGTC GACCTCCGCA GCCTGTTCCT GTTCAGCTAC 2220
   CACCACCGCG ACCTGCTGCT GATCGCCGCC CGCATCGTGG AACTCCTAGG CCGCCGCGGC 2280
   TGGGAGGTGC TGAAGTACTG GTGGAACCTC CTCCAGTATT GGAGCCAGGA GCTGAAGTCC 2340
   AGCGCCGTGA GCCTGCTGAA CGCCACCGCC ATCGCCGTGG CCGAGGGCAC CGACCGCGTG 2400
   ATCGAGGTGC TCCAGAGGGC CGGGAGGGCG ATCCTGCACA TCCCCACCCG CATCCGCCAG 2460
   GGGCTCGAGA GGGCGCTGCT G 2481
(2) INFORMATION FOR SEQ ID NO:36:
   (i) SEQUENCE CHARACTERISTICS:
      (A) LENGTH: 486 base pairs
      (B) TYPE: nucleic acid
      (C) STRANDEDNESS: single
      (D) TOPOLOGY: linear
   (xi) SEQUENCE DESCRIPTION: SEQ ID NO:36:
   ATGAATCCAG TAATAAGTAT AACATTATTA TTAAGTGTAT TACAAATGAG TAGAGGACAA     60
   AGAGTAATAA GTTTAACAGC ATGTTTAGTA AATCAAAATT TGAGATTAGA TTGTAGACAT    120
   GAAAATAATA CACCTTTGCC AATACAACAT GAATTTTCAT TAACGCGTGA AAAAAAAAAA    180
   CATGTATTAA GTGGAACATT AGGAGTACCA GAACATACAT ATAGAAGTAG AGTAAATTTG    240
   TTTAGTGATA GATTCATAAA AGTATTAACA TTAGCAAATT TTACAACAAA AGATGAAGGA    300
   GATTATATGT GTGAGCTCAG AGTAAGTGGA CAAAATCCAA CAAGTAGTAA TAAAACAATA    360
   AATGTAATAA GAGATAAATT AGTAAAATGT GGAGGAATAA GTTTATTAGT ACAAAATACA    420
   AGTTGGTTAT TATTATTATT ATTAAGTTTA AGTTTTTTAC AAGCAACAGA TTTTATAAGT    480
   TTATGA                                                               486
(2) INFORMATION FOR SEQ ID NO:37:
   (i) SEQUENCE CHARACTERISTICS:
      (A) LENGTH: 485 base pairs
      (B) TYPE: nucleic acid
      (C) STRANDEDNESS: single
      (D) TOPOLOGY: linear
   (xi) SEQUENCE DESCRIPTION: SEQ ID NO:37:
   ATGAACCCAG TCATCAGCAT CACTCTCCTG CTTTCAGTCT TGCAGATGTC CCGAGGACAG     60
   AGGGTGATCA GCCTGACAGC CTGCCTGGTG AACAGAACCT TCGACTGGAC TGCCGTCATG    120
   AGAATAACAC CAACTTGCCC ATCCAGCATG AGTTCAGCCT GACCCGAGAG AAGAAGAAGC    180
   ACGTGCTGTC AGGCACCCTG GGGGTTCCCG AGCACACTTA CCGCTCCCGC GTCAACCTTT    240
   TCAGTGACCG CTTTATCAAG GTCCTTACTC TAGCCAACTT GACCACCAAG GATGAGGGCG    300
   ACTACATGTG TGAACTTCGA GTCTCGGGCC AGAATCCCAC AAGCTCCAAT AAAACTATCA    360
   ATGTGATCAG AGACAAGCTG GTCAAGTGTG GTGGCATAAG CCTGCTGGTT CAAAACACTT    420
   CCTGGCTGCT GCTGCTCCTG CTTTCCCTCT CCTTCCTCCA AGCCACGGAC TTCATTTCTC    480
   TGTGA                                                                485

## Description

### Field of the Invention

The invention concerns genes and methods for expressing HIV proteins at high levels in eukaryotic cells.

### Background of the Invention

Expression of eukaryotic gene products in prokaryotes is sometimes limited by the presence of codons that are infrequently used in E. coli. Expression of such genes can be enhanced by systematic substitution of the endogenous codons with codons overrepresented in highly expressed prokaryotic genes (Robinson et al. 1984). It is commonly supposed that rare codons cause pausing of the ribosome, which leads to a failure to complete the nascent polypeptide chain and a uncoupling of transcription and translation. The mRNA 3' end of the stalled ribosome is exposed to cellular ribonucleases, which decreases the stability of the transcript.

### Summary of the Invention

The invention features method for preparing a synthetic gene, and a synthetic gene obtainable by this method according to the appended claims.

Preferred codons are: Ala (gcc); Arg (cgc); Asn (aac); Asp (gac) Cys (tgc); Gln (cag); Gly (ggc); His (cac); Ile (atc); Leu (ctg); Lys (aag); Pro (ccc); Phe (ttc); Ser (agc); Thr (acc); Tyr (tac); and Val (gtg). Less preferred codons are: Gly (ggg); Ile (att); Leu (ctc); Ser (tcc); Val (gtc). All codons which do not fit the description of preferred codons or less preferred codons are non-preferred codons.

In preferred embodiments, the synthetic gene is capable of expressing said mammalian protein at a level which is at least 150%, 200%, 500%, 1,000%, or 10,000% of that expressed by said natural gene in an in vitro mammalian cell culture system under identical conditions (i.e., same cell type, same culture conditions, same expression vector).

Suitable cell culture systems for measuring expression of the synthetic gene and corresponding natural gene are described below. Other suitable expression systems employing mammalian cells are well known to those skilled in the art and are described in, for example, the standard molecular biology reference works noted below. Vectors suitable for expressing the synthetic and natural genes are described below and in the standard reference works described below. By "expression" is meant protein expression. Expression can be measured using an antibody specific for the protein of interest. Such antibodies and measurement techniques are well known to those skilled in the art. By "natural gene" is meant the gene sequence which naturally encodes the protein.

In other preferred embodiments at least 10%, 20%, 30%, 40%, 50%, 60%, 70%, 80%, or 90% of the codons in the natural gene are non-preferred codons.

In a preferred embodiment the protein is an HIV protein. In other preferred embodiments the protein is gag, pol, env, gp120, or gp160.

Under some circumstances (e.g., to permit introduction of a restriction site) it may be desirable to replace a non-preferred codon with a less preferred codon rather than a preferred codon.

It is not necessary to replace all less preferred or non-preferred codons with preferred codons. Increased expression can be accomplished even with partial replacement.

In other preferred embodiments the invention features vectors (including expression vectors) comprising the synthetic gene.

By "vector" is meant a DNA molecule, derived, e.g., from a plasmid, bacteriophage, or mammalian or insect virus, into which fragments of DNA may be inserted or cloned. A vector will contain one or more unique restriction sites and may be capable of autonomous replication in a defined host or vehicle organism such that the cloned sequence is reproducible. Thus, by "expression vector" is meant any autonomous element capable of directing the synthesis of a protein. Such DNA expression vectors include mammalian plasmids and viruses.

In constructing the synthetic genes of the invention it may be desirable to avoid CpG sequences as these sequences may cause gene silencing.

The codon bias present in the HIV gp120 envelope gene is also present in the gag and pol proteins. Thus, replacement of a portion of the non-preferred and less preferred codons found in these genes with preferred codons should produce a gene capable of higher level expression. A large fraction of the codons in the human genes encoding Factor VIII and Factor IX are non-preferred codons or less preferred codons. Replacement of a portion of these codons with preferred codons should yield genes capable of higher level expression in mammalian cell culture. Conversely, it may be desirable to replace preferred codons in a naturally occurring gene with less-preferred codons as a means of lowering expression.

Standard reference works describing the general principles of recombinant DNA technology include Watson, J.D. et al., Molecular Biology of the Gene, Volumes I and II, the Benjamin/Cummings Publishing Company, Inc., publisher, Menlo Park, CA (1987); Darnell, J.E. et al., Molecular Cell Biology, Scientific American Books, Inc., Publisher, New York, N.Y. (1986); Old, R.W., et al., Principles of Gene Manipulation: An Introduction to Genetic Engineering, 2d edition, University of California Press, publisher, Berkeley, CA (1981); Maniatis, T., et al., Molecular Cloning: A Laboratory Manual, 2nd Ed. Cold Spring Harbor Laboratory, publisher, Cold Spring Harbor, NY (1989); and Current Protocols in Molecular Biology. Ausubel et al., Wiley Press, New York, NY (1989).

### Detailed Description

### Description of the Drawings

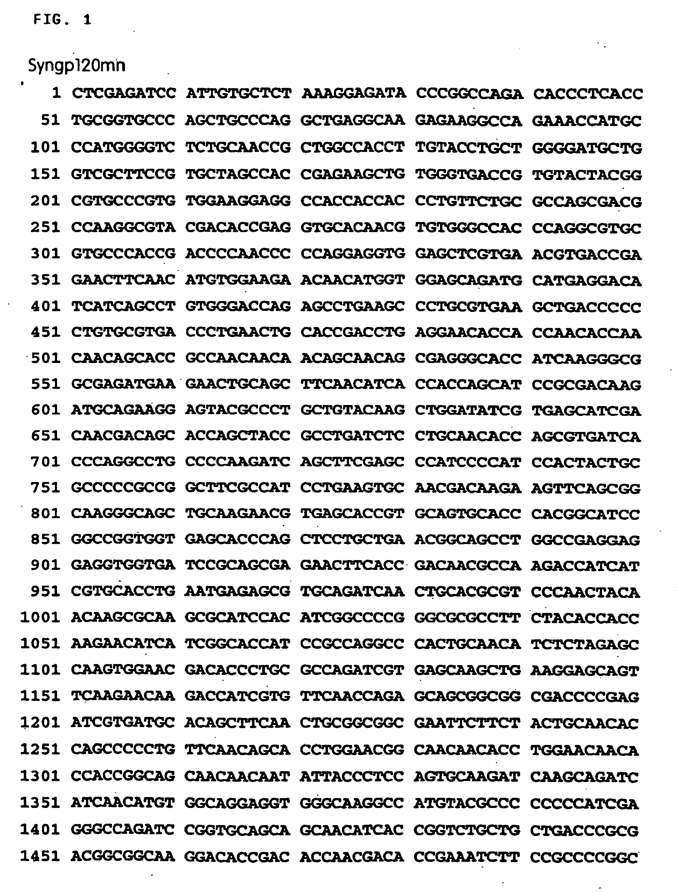

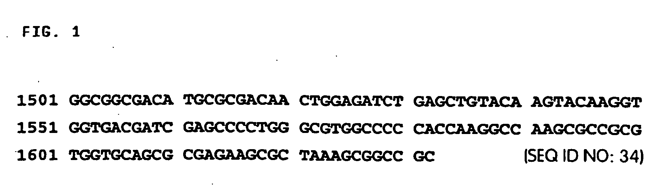

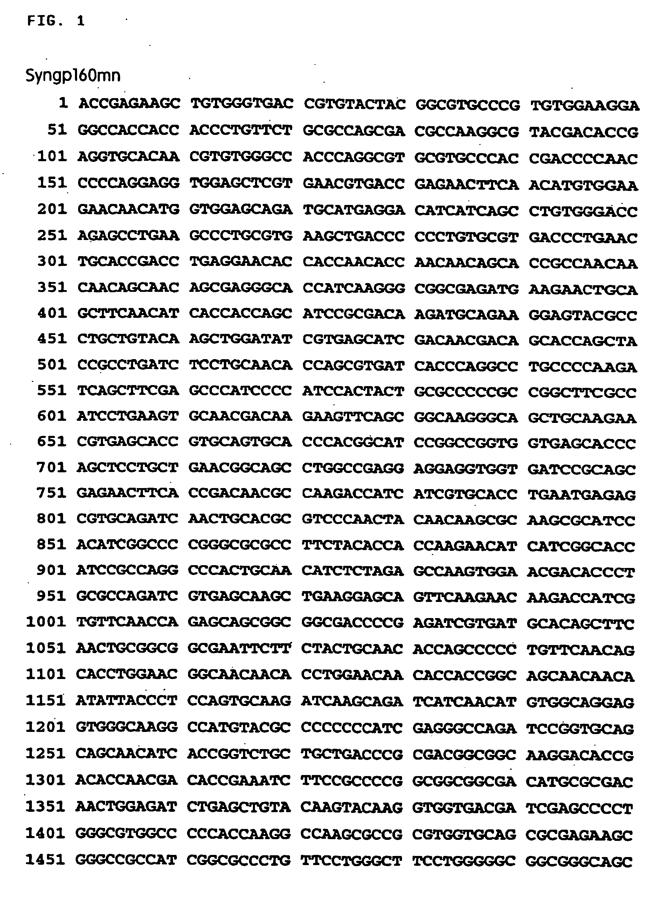

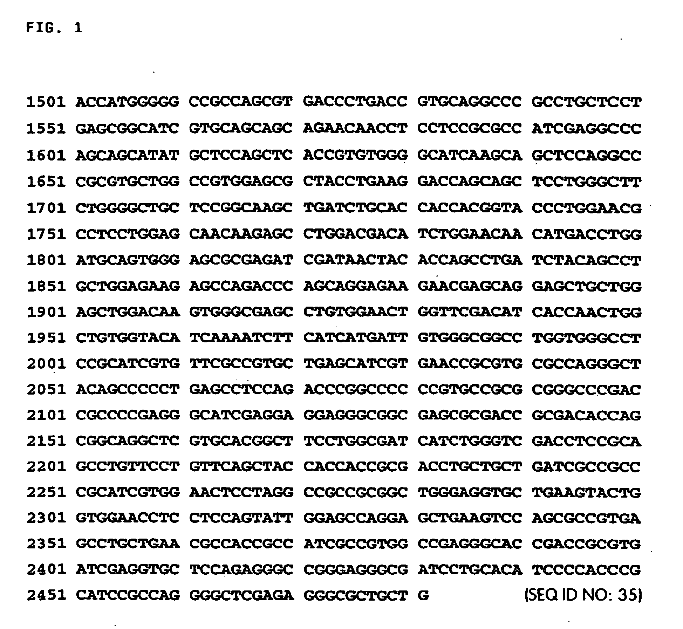

Figure 1 depicts the sequence of the synthetic gp120 (SEQ ID NO: 34) and a synthetic gp160 (SEQ ID NO: 35) gene in which codons have been replaced by those found in highly expressed human genes.

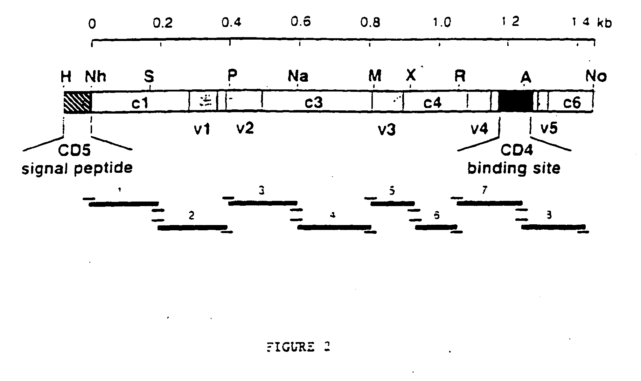

Figure 2 is a schematic drawing of the synthetic gp120 (HIV-1 MN) gene. The shaded portions marked v1 to v5 indicate hypervariable regions. The filled box indicates the CD4 binding site. A limited number of the unique restriction sites ares shown: H (Hind3), Nh (Nhe1), P (Pst1), Na (Nae1), M (Mlul), R (EcoR1), A (Age1) and No (Not1). The chemically synthesized DNA fragments which served as PCR templates are shown below the gp120 sequence, along with the locations of the primers used for their amplification.

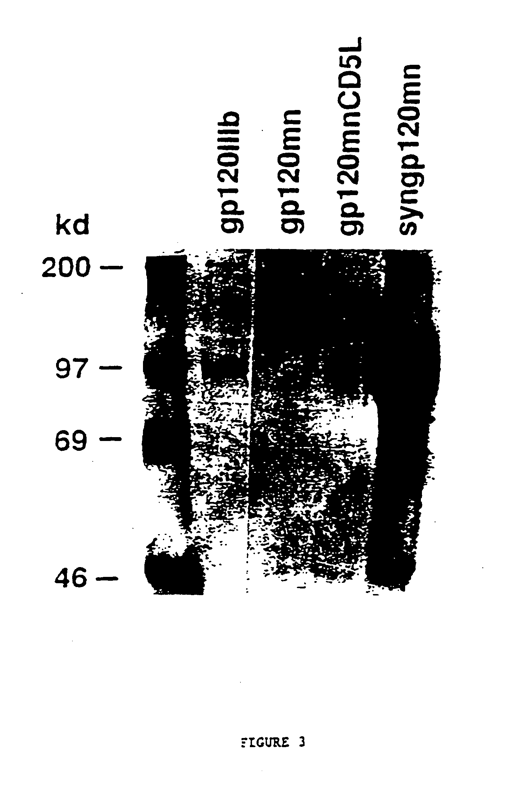

Figure 3 is a photograph of the results of transient transfection assays used to measure gp120 expression. Gel electrophoresis of immunoprecipitated supernatants of 293T cells transfected with plasmids expressing gp120 encoded by the IIIB isolate of HIV-1 (gp120IIIb), by the MN isolate (gp120mn), by the MN isolate modified by substitution of the endogenous leader peptide with that of the CD5 antigen (gp120mnCD5L), or by the chemically synthesized gene encoding the MN variant with the human CDSLeader (syngp120mn). Supernatants were harvested following a 12 hour labeling period 60 hours post-transfection and immunoprecipitated with CD4:IgG1 fusion protein and protein A sepharose.

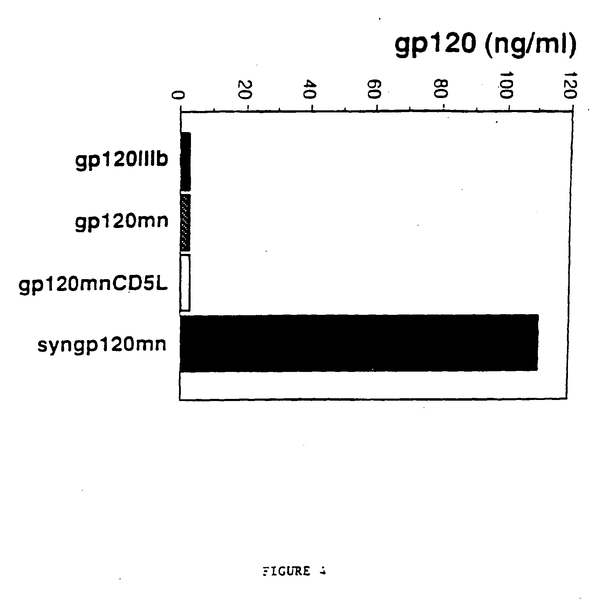

Figure 4 is a graph depicting the results of ELISA assays used to measure protein levels in supernatants of transiently transfected 293T cells. Supernatants of 293T cells transfected with plasmids expressing gp120 encoded by the IIIB isolate of HIV-1 (gp120 IIIb), by the MN isolate (gp120mn), by the MN isolate modified by substitution of the endogenous leader peptide with that of CD5 antigen (gp120mn CD5L), or by the chemically synthesized gene encoding the MN variant with human CDS leader (syngp120mn) were harvested after 4 days and tested in a gp120/CD4 ELISA. The level of gp120 is expressed in ng/ml.

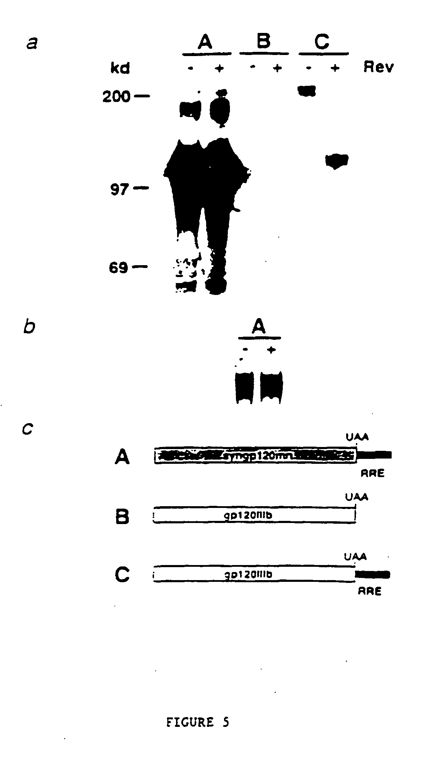

Figure 5, panel A is a photograph of a gel illustrating the results of a immunoprecipitation assay used to measure expression of the native and synthetic gp120 in the presence of rev in trans and the RRE in cis. In this experiment 293T cells were transiently transfected by calcium phosphate coprecipitation of 10 µg of plasmid expressing: (A) the synthetic gp120MN sequence and RRE in cis, (B) the gp120 portion of HIV-1 IIIB, (C) the gp120 portion of HIV-1 IIIB and RRE in cis, all in the presence or absence of rev expression. The RRE constructs qp120IIIbRRE and syngpl20mnRRE were generated using an Eagl/Hpal RRE fragment cloned by PCR from a HIV-1 HXB2 proviral clone. Each gp120 expression plasmid was cotransfected with 10 *µ*g of either pCMVrev or CDM7 plasmid DNA. Supernatants were harvested 60 hours post transfection, immunoprecipitated with CD4:IgG fusion protein and protein A agarose, and run on a 7% reducing SDS-PAGE. The gel exposure time was extended to allow the induction of gp120IIIbrre by rev to be demonstrated. Figure 5, panel B is a shorter exposure of a similar experiment in which syngp120mnrre was cotransfected with or without pCMVrev. Figure 5, panel C is a schematic diagram of the constructs used in panel A.

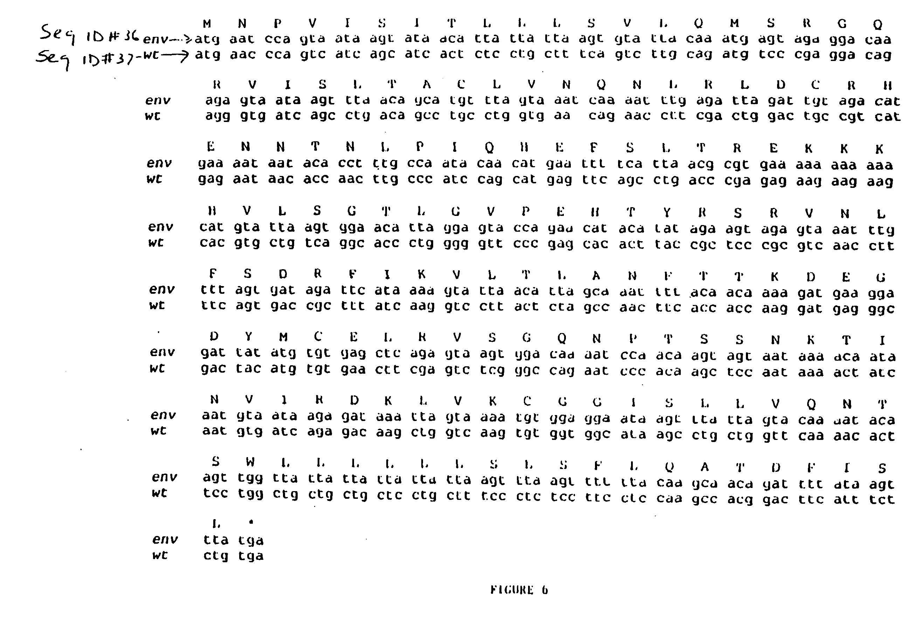

Figure 6 is a comparison of the sequence of the wildtype rat THY-1 gene (wt) (SEQ. ID. NO: 37) and a synthetic rat THY-1 gene (env) (SEQ. ID. NO: 36) constructed by chemical synthesis and having the most prevalent codons found in the HIV-1 env gene.

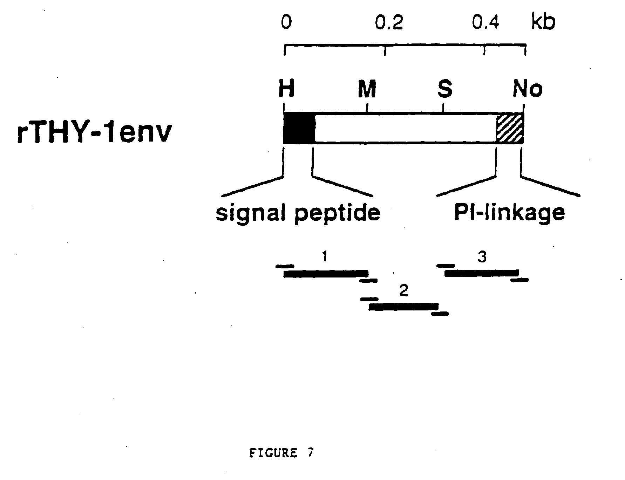

Figure 7 is a schematic diagram of the synthetic ratTHY-1 gene. The solid black box denotes the signal peptide. The shaded box denotes the sequences in the precursor which direct the attachment of a phophatidylinositol glycan anchor. Unique restriction sites used for assembly of the THY-1 constructs are marked H (Hind3), M (Mlul), S (Sac1) and No (Not1). The position of the synthetic oligonucleotides employed in the construction are shown at the bottom of the figure.

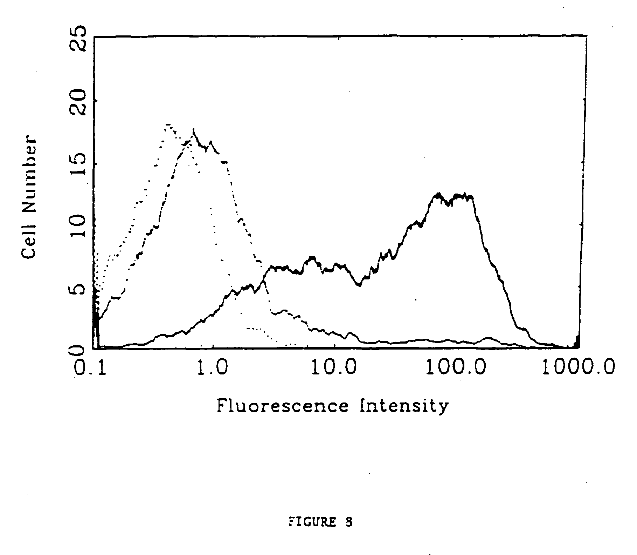

Figure 8 is a graph depicting the results of flow cytometry analysis. In this experiment 293T cells transiently transfected with either wildtype rat THY-1 (dark line), ratTHY-1 with envelope codons (light line) or vector only (dotted line). 293T cells were transfected with the different expression plasmids by calcium phosphate coprecipitation and stained with anti-ratTHY-1 monoclonal antibody OX7 followed by a polyclonal FITC- conjugated anti-mouse IgG antibody 3 days after transfection.

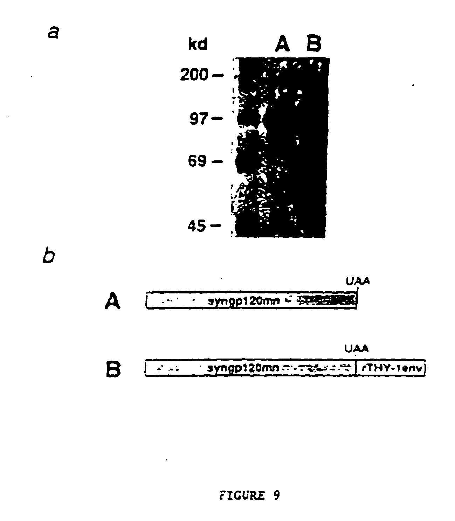

Figure 9, panel A is a photograph of a gel illustrating the results of immunoprecipitation analysis of supernatants of human 293T cells transfected with either syngp120mn (A) or a construct syngp120mn.rTHY-lenv which has the rTHY-lenv gene in the 3' untranslated region of the syngp120mn gene (B). The syngp120mn.rTHY-lenv construct was generated by inserting a Not1 adapter into the blunted Hind3 site of the rTHY-lenv plasmid. Subsequently, a 0.5 kb Not1 fragment containing the rTHY-lenv gene was cloned into the Not1 site of the syngp120mn plasmid and tested for correct orientation. Supernatants of 35S labelled cells were harvested 72 hours post transfection, precipitated with CD4:IgG fusion protein and protein A agarose, and run on a 7% reducing SDS-PAGE. Figure 9, panel B is a schematic diagram of the constructs used in the experiment depicted in panel A of this figure.

### Description of the Preferred Embodiments

### Construction of a Synthetic gp120 Gene Having Codons Found in Highly Expressed Human Genes

A codon frequency table for the envelope precursor of the LAV subtype of HIV-1 was generated using software developed by the University of Wisconsin Genetics Computer Group. The results of that tabulation are contrasted in Table 1 with the pattern of codon usage by a collection of highly expressed human genes. For any amino acid encoded by degenerate codons, the most favored codon of the highly expressed genes is different from the most favored codon of the HIV envelope precursor. Moreover a simple rule describes the pattern of favored envelope codons wherever it applies: preferred codons maximize the number of
adenine residues in the viral RNA. In all cases but one this means that the codon in which the third position is A is the most frequently used. In the special case of serine, three codons equally contribute one A residue to the mRNA; together these three comprise 85% of the codons actually used in envelope transcripts. A particularly striking example of the A bias is found in the codon choice for arginine, in which the AGA triplet comprises 88% of all codons. In addition to the preponderance of A residues, a marked preference is seen for uridine among degenerate codons whose third residue must be a pyrimidine. Finally, the inconsistencies among the less frequently used variants can be accounted for by the observation that the dinucleotide CpG is underrepresented; thus the third position is less likely to be G whenever the second position is C, as in the codons for alanine, proline, serine and threonine; and the CGX triplets for arginine are hardly used at all.

**TABLE 1: Codon Frequency in the HIV-1 IIIb env gene and in highly expressed human genes.**

| | | **High** | **Env** | | | **High** | **Env** |
|---|---|---|---|---|---|---|---|
| **Ala** | | | | **Cys** | | | |
| GC | C | 53 | 27 | TG | C | 68 | 16 |
| | T | 17 | 18 | | T | 32 | 84 |
| | A | 13 | 50 | | | | |
| | G | 17 | 5 | **Gln** | | | |
| | | | | CA | A | 12 | 55 |
| **Arg** | | | | | G | 88 | 45 |
| CG | C | 37 | 0 | | | | |
| | T | 7 | 4 | **Glu** | | | |
| | A | 6 | 0 | GA | A | 25 | 67 |
| | G | 21 | 0 | | G | 75 | 33 |
| AG | A | 10 | 88 | | | | |
| | G | 18 | 8 | **Gly** | | | |
| | | | | GG | C | 50 | 6 |
| **Asn** | | | | | T | 12 | 13 |
| AA | C | 78 | 30 | | A | 14 | 53 |
| | T | 22 | 70 | | G | 24 | 28 |
| **Asp** | | | | **His** | | | |
| GA | C | 75 | 33 | CA | C | 79 | 25 |
| | T | 25 | 67 | | T | 21 | 75 |
| | | | | **Ile** | | | |
| | | | | AT | C | 77 | 25 |
| | | | | | T | 18 | 31 |
| | | | | | A | 5 | 44 |
| **Leu** | | | | **Ser** | | | |
| CT | C | 26 | 10 | TC | C | 28 | 8 |
| | T | 5 | 7 | | T | 13 | 8 |
| | A | 3 | 17 | | A | 5 | 22 |
| | G | 58 | 17 | | G | 9 | 0 |
| TT | A | 2 | 30 | AG | C | 34 | 22 |
| | G | 6 | 20 | | T | 10 | 41 |
| **Lys** | | | | **Thr** | | | |
| AA | A | 18 | 68 | AC | C | 57 | 20 |
| | G | 82 | 32 | | T | 14 | 22 |
| | | | | | A | 14 | 51 |
| | | | | | G | 15 | 7 |
| **Pro** | | | | **Tyr** | | | |
| CC | C | 48 | 27 | TA | C | 74 | 8 |
| | T | 19 | 14 | | T | 26 | 92 |
| | A | 16 | 55 | | | | |
| | G | 17 | 5 | | | | |
| **Phe** | | | | **Val** | | | |
| TT | C | 80 | 26 | GT | C | 25 | 12 |
| | T | 20 | 74 | | T | 7 | 9 |
| | | | | | A | 5 | 62 |
| | | | | | G | 64 | 18 |
| Codon frequency was calculated using the GCG program established the University of Wisconsin Genetics Computer Group. Numbers represent the percentage of cases in which the particular codon is used. Codon usage frequencies of envelope genes of other HIV-1 virus isolates are comparable and show a similar bias. | | | | | | | |

In order to produce a gp120 gene capable of high level expression in mammalian cells, a synthetic gene encoding the gp120 segment of HIV-1 was constructed (syngp120mn), based on the sequence of the most common North American subtype, HIV-1 MN (Shaw et al. 1984; Gallo et al. 1986). In this synthetic gp120 gene nearly all of the native codons have been systematically replaced with codons most frequently used in highly expressed human genes (FIG. 1). This synthetic gene was assembled from chemically synthesized oligonucleotides of 150 to 200 bases in length. If oligonucleotides exceeding 120 to 150 bases are chemically synthesized, the percentage of full-length product can be low, and the vast excess of material consists of shorter oligonucleotides. Since these shorter fragments inhibit cloning and PCR procedures, it can be very difficult to use oligonucleotides exceeding a certain length. In order to use crude synthesis material without prior purification, single-stranded oligonucleotide pools were PCR amplified before cloning. PCR products were purified in agarose gels and used as templates in the next PCR step. Two adjacent fragments could be co-amplified because of overlapping sequences at the end of either fragment. These fragments, which were between 350 and 400 bp in size, were subcloned into a pCDM7-derived plasmid containing the leader sequence of the CD5 surface molecule followed by a Nhe1/Pst1/Mlu1/EcoR1/BamH1 polylinker. Each of the restriction enzymes in this polylinker represents a site that is present at either the 5' or 3' end of the PCR-generated fragments. Thus, by sequential subcloning of each of the 4 long fragments, the whole gp120 gene was assembled. For each fragment 3 to 6 different clones were subcloned and sequenced prior to assembly. A schematic drawing of the method used to construct the synthetic gp120 is shown in FIG. 2. The sequence of the synthetic gp120 gene (and a synthetic gp160 gene created using the same approach) is presented in FIG. 1.

The mutation rate was considerable. The most commonly found mutations were short (1 nucleotide) and long (up to 30 nucleotides) deletions. In some cases it was necessary to exchange parts with either synthetic adapters or pieces from other subclones without mutation in that particular region. Some deviations from strict adherence to optimized codon usage were made to accommodate the introduction of restriction sites into the resulting gene to facilitate the replacement of various segments (FIG. 2). These unique restriction sites were introduced into the gene at approximately 100 bp intervals. The native HIV leader sequence was exchanged with the highly efficient leader peptide of the human CD5 antigen to facilitate secretion. The plasmid used for construction is a derivative of the mammalian expression vector pCDM7 transcribing the inserted gene under the control of a strong human CMV immediate early promoter.

To compare the wild-type and synthetic gp120 coding sequences, the synthetic gp120 coding sequence was inserted into a mammalian expression vector and tested in transient transfection assays. Several different native gp120 genes were used as controls to exclude variations in expression levels between different virus isolates and artifacts induced by distinct leader sequences. The gp120 HIV IIIb construct used as control was generated by PCR using a Sal1/Xho1 HIV-1 HXB2 envelope fragment as template. To exclude PCR induced mutations a Kpn1/Ear1 fragment containing approximately 1.2 kb of the gene was exchanged with the respective sequence from the proviral clone. The wildtype gp120mn constructs used as controls were cloned by PCR from HIV-1 MN infected C8166 cells (AIDS Repository, Rockville, MD) and expressed gp120 either with a native envelope or a CD5 leader sequence. Since proviral clones were not available in this case, two clones of each construct were tested to avoid PCR artifacts. To determine the amount of secreted gp120 semi-quantitatively supernatants of 293T cells transiently transfected by calcium phosphate coprecipitation were immunoprecipitated with soluble CD4:immunoglobulin fusion protein and protein A sepharose.

The results of this analysis (FIG. 3) show that the synthetic gene product is expressed at a very high level compared to that of the native gp120 controls. The molecular weight of the synthetic gp120 gene was comparable to control proteins (FIG. 3) and appeared to be in the range of 100 to 110 kd. The slightly faster migration can be explained by the fact that in some tumor cell lines like 293T glycosylation is either not complete or altered to some extent.

To compare expression more accurately gp120 protein levels were quantitated using a gp120 ELISA with CD4 in the demobilized phase. This analysis shows (FIG. 4) that ELISA data were comparable to the immunoprecipitation data, with a gp120 concentration of approximately 125 ng/ml for the synthetic gp120 gene, and less than the background cutoff (5 ng/ml) for all the native gp120 genes. Thus, expression of the synthetic gp120 gene appears to be at least one order of magnitude higher than wildtype gp120 genes. In the experiment shown the increase was at least 25 fold.

### The Role of rev in gp120 Expression

Since rev appears to exert its effect at several steps in the expression of a viral transcript, the possible role of non-translational effects in the improved expression of the synthetic gp120 gene was tested. First, to rule out the possibility that negative signals elements conferring either increased mRNA degradation or nucleic retention were eliminated by changing the nucleotide sequence, cytoplasmic mRNA levels were tested. Cytoplasmic RNA was prepared by NP40 lysis of transiently transfected 293T cells and subsequent elimination of the nuclei by centrifugation. Cytoplasmic RNA was subsequently prepared from lysates by multiple phenol extractions and precipitation, spotted on nitrocellulose using a slot blot apparatus, and finally hybridized with an envelope-specific probe.

Briefly, cytoplasmic mRNA 293 cells transfected with CDM&, gp120 IIIB, or syngp120 was isolated 36 hours post transfection. Cytoplasmic RNA of Hela cells infected with wildtype vaccinia virus or recombinant virus expressing gp120 IIIb or the synthetic gp120 gene was under the control of the 7.5 promoter was isolated 16 hours post infection. Equal amounts were spotted on nitrocellulose using a slot blot device and hybridized with randomly labelled 1.5 kb gp120IIIb and syngp120 fragments or human beta-actin. RNA expression levels were quantitated by scanning the hybridized membranes with a phospoimager. The procedures used are described in greater detail below.

This experiment demonstrated that there was no significant difference in the mRNA levels of cells transfected with either the native or synthetic gp120 gene. In fact, in some experiments cytoplasmic mRNA level of the synthetic gp120 gene was even lower than that of the native gp120 gene.

These data were confirmed by measuring expression from recombinant vaccinia viruses. Human 293 cells or Hela cells were infected with vaccinia virus expressing wildtype gp120 IIIb or syngp120mn at a multiplicity of infection of at least 10. Supernatants were harvested 24 hours post infection and immunoprecipitated with CD4:immunoglobin fusion protein and protein A sepharose. The procedures used in this experiment are described in greater detail below.

This experiment showed that the increased expression of the synthetic gene was still observed when the endogenous gene product and the synthetic gene product were expressed from vaccinia virus recombinants under the control of the strong mixed early and late 7.5k promoter. Because vaccinia virus mRNAs are transcribed and translated in the cytoplasm, increased expression of the synthetic envelope gene in this experiment cannot be attributed to improved export from the nucleus. This experiment was repeated in two additional human cell types, the kidney cancer cell line 293 and HeLa cells. As with transfected 293T cells, mRNA levels were similar in 293 cells infected with either recombinant vaccinia virus.

### Codon Usage in Lentivirus

Because it appears that codon usage has a significant impact on expression in mammalian cells, the codon frequency in the envelope genes of other retroviruses was examined. This study found no clear pattern of codon preference between retroviruses in general. However, if viruses from the lentivirus genus, to which HIV-1 belongs to, were analyzed separately, codon usage bias almost identical to that of HIV-1 was found. A codon frequency table from the envelope glycoproteins of a variety of (predominantly type C) retroviruses excluding the lentiviruses was prepared, and compared a codon frequency table created from the envelope sequences of four lentiviruses not closely related to HIV-1 (caprine arthritis encephalitis virus, equine infectious anemia virus, feline immunodeficiency virus, and visna virus) (Table 2). The codon usage pattern for lentiviruses is strikingly similar to that of HIV-1, in all cases but one, the preferred codon for HIV-1 is the same as the preferred codon for the other lentiviruses. The exception is proline, which is encoded by CCT in 41% of non-HIV lentiviral envelope residues, and by CCA in 40% of residues, a situation which clearly also reflects a significant preference for the triplet ending in A. The pattern of codon usage by the non-lentiviral envelope proteins does not show a similar predominance of A residues, and is also not as skewed toward third position C and G residues as is the codon usage for the highly expressed human genes. In general non-lentiviral retroviruses appear to exploit the different codons more equally, a pattern they share with less highly expressed human genes.

**TABLE 2: Codon frequency in the envelope gene of lentiviruses (lenti) and non-lentiviral retroviruses (other).**

| | | **other Lenti** | | | | **Other Lenti** | |
|---|---|---|---|---|---|---|---|
| **Ala** | | | | **Cys** | | | |
| GC | C | 45 | 13 | TG | C | 53 | 21 |
| | T | 26 | 37 | | T | 47 | 79 |
| | A | 20 | 46 | | | | |
| | G | 9 | 3 | **Gln** | | | |
| | | | | CA | A | 52 | 69 |
| **Arg** | | | | | G | 48 | 31 |
| CG | C | 14 | 2 | | | | |
| | T | 6 | 3 | **Glu** | | | |
| | A | 16 | 5 | GA | A | 57 | 68 |
| | G | 17 | 3 | | G | 43 | 32 |
| AG | A | 31 | 51 | | | | |
| | G | 15 | 26 | **Gly** | | | |
| | | | | GG | C | 21 | 8 |
| **Asn** | | | | | T | 13 | 9 |
| AA | C | 49 | 31 | | A | 37 | 56 |
| | T | 51 | 69 | | G | 29 | 26 |
| **Asp** | | | | **His** | | | |
| GA | C | 55 | 33 | CA | C | 51 | 38 |
| | T | 51 | 69 | | T | 49 | 62 |
| | | | | **Ile** | | | |
| | | | | AT | C | 38 | 16 |
| | | | | | T | 31 | 22 |
| | | | | | A | 31 | 61 |
| **Leu** | | | | **Ser** | | | |
| CT | C | 22 | 8 | TC | C | 38 | 10 |
| | T | 14 | 9 | | T | 17 | 16 |
| | A | 21 | 16 | | A | 18 | 24 |
| | G | 19 | 11 | | G | 6 | 5 |
| TT | A | 15 | 41 | AG | C | 13 | 20 |
| | G | 10 | 16 | | T | 7 | 25 |
| **Lys** | | | | **Thr** | | | |
| AA | A | 60 | 63 | AC | C | 44 | 18 |
| | G | 40 | 37 | | T | 27 | 20 |
| | | | | | A | 19 | 55 |
| **Pro** | | | | | G | 10 | 8 |
| CC | C | 42 | 14 | | | | |
| | T | 30 | 41 | **Tyr** | | | |
| | A | 20 | 40 | TA | C | 48 | 28 |
| | G | 7 | 5 | | T | 52 | 72 |
| **Phe** | | | | **Val** | | | |
| TT | C | 52 | 25 | GT | C | 36 | 9 |
| | T | 48 | 75 | | T | 17 | 10 |
| | | | | | A | 22 | 54 |
| | | | | | G | 25 | 27 |
| Codon frequency was calculated using the GCG program established by the University of Wisconsin Genetics Computer Group. Numbers represent the percentage in which a particular codon is used. Codon usage of non-lentiviral retroviruses was compiled from the envelope precursor sequences of bovine leukemia virus feline leukemia virus, human T-cell leukemia virus type I, human T-cell lymphotropic virus type II, the mink cell focus-forming isolate of murine leukemia virus (MuLV), the Rauscher spleen focus-forming isolate, the 10A1 isolate, the 4070A amphotropic isolate and the myeloproliferative leukemia virus isolate, and from rat leukemia virus, simian sarcoma virus, simian T-cell leukemia virus, leukemogenic retrovirus T1223/B and gibbon ape leukemia virus. The codon frequency tables for the non-HIV, non-SIV lentiviruses were compiled from the envelope precursor sequences for caprine arthritis encephalitis virus, equine infectious anemia virus, feline immunodeficiency virus, and visna virus. | | | | | | | |

In addition to the prevalence of A containing codons, lentiviral codons adhere to the HIV pattern of strong CpG underrepresentation, so that the third position for alanine, proline, serine and threonine triplets is rarely G. The retroviral envelope triplets show a similar, but less pronounced, underrepresentation of CpG. The most obvious difference between lentiviruses and other retroviruses with respect to CpG prevalence lies in the usage of the CGX variant of arginine triplets, which is reasonably frequently represented among the retroviral envelope coding sequences, but is almost never present among the comparable lentivirus sequences.

### Differences in rev Dependence Between Native and Synthetic gp120

To examine whether regulation by rev is connected to HIV-1 codon usage, the influence of rev on the expression of both native and synthetic gene was investigated. Since regulation by rev requires the rev-binding site RRE in cis, constructs were made in which this binding site was cloned into the 3' untranslated region of both the native and the synthetic gene. These plasmids were co-transfected with rev or a control plasmid in trans into 293T cells, and gp120 expression levels in supernatants were measured semiquantitatively by immunoprecipitation. The procedures used in this experiment are described in greater detail below.

As shown in FIG. 5, panels A and B, rev upregulates the native gp120 gene, but has no effect on the expression of the synthetic gp120 gene. Thus, the action of rev is not apparent on a substrate which lacks the coding sequence of endogenous viral envelope sequences.

### Expression of a synthetic rat THY-1 gene with HIV envelope codons

The above-described experiment suggest that in fact "envelope sequences" have to be present for rev regulation. In order to test this hypothesis, a synthetic version of the gene encoding the small, typically highly expressed cell surface protein, rat THY-1 antigen, was prepared. The synthetic version of the rat THY-1 gene was designed to have a codon usage like that of HIV gp120. In designing this synthetic gene AUUUA sequences, which are associated with mRNA instability, were avoided. In addition, two restriction sites were introduced to simplify manipulation of the resulting gene (FIG. 6). This synthetic gene with the HIV envelope codon usage (rTHY-1env) was generated using three 150 to 170 mer oligonucleotides (FIG. 7). In contrast to the syngp120mn gene, PCR products were directly cloned and assembled in pUC12, and subsequently cloned into pCDM7.

Expression levels of native rTHY-1 and rTHY-1 with the HIV envelope codons were quantitated by immunofluorescence of transiently transfected 293T cells. FIG 8 shows that the expression of the native THY-1 gene is almost two orders of magnitude above the background level of the control transfected cells (pCDM7). In contrast, expression of the synthetic rat THY-1 is substantially lower than that of the native gene (shown by the shift to of the peak towards a lower channel number).

To prove that no negative sequence elements promoting mRNA degradation were inadvertently introduced, a construct was generated in which the rTHY-lenv gene was cloned at the 3' end of the synthetic gp120 gene (FIG. 9, panel B). In this experiment 293T cells were transfected with either the syngp120mn gene or the syngp120/rat THY-1 env fusion gene (syngp120mn.rTHY-lenv). Expression was measured by immunoprecipitation with CD4:IgG fusion protein and protein A agarose. The procedures used in this experiment are described in greater detail below.

Since the synthetic gp120 gene has an UAG stop codon, rTHY-1env is not translated from this transcript. If negative elements conferring enhanced degradation were present in the sequence, gp120 protein levels expressed from this construct should be decreased in comparison to the syngp120mn construct without rTHY-1env. FIG. 9, panel A, shows that the expression of both constructs is similar, indicating that the low expression must be linked to translation.

### Rev-dependent expression of synthetic rat THY-1 gene with envelope codons

To explore whether rev is able to regulate expression of a rat THY-1 gene having env codons, a construct was made with a rev-binding site in the 3' end of the rTHYlenv open reading frame. To measure rev-responsiveness of the a rat THY-lenv construct having a 3' RRE, human 293T cells were cotransfected ratTHY-lenvrre and either CDM7 or pCMVrev. At 60 hours post transfection cells were detached with 1 mM EDTA in PBS and stained with the OX-7 anti rTHY-1 mouse monoclonal antibody and a secondary FITC-conjugated antibody. Fluorescence intensity was measured using a EPICS XL cytofluorometer. These procedures are described in greater detail below.

In repeated experiments, a slight increase of rTHY-lenv expression was detected if rev was cotransfected with the rTHY-lenv gene. To further increase the sensitivity of the assay system a construct expressing a secreted version of rTHY-lenv was generated. This construct should produce more reliable data because the accumulated amount of secreted protein in the supernatant reflects the result of protein production over an extended period, in contrast to surface expressed protein, which appears to more closely reflect the current production rate. A gene capable of expressing a secreted form was prepared by PCR using forward and reverse primers annealing 3' of the endogenous leader sequence and 5' of the sequence motif required for phosphatidylinositol glycan anchorage respectively. The PCR product was cloned into a plasmid which already contained a CD5 leader sequence, thus generating a construct in which the membrane anchor has been deleted and the leader sequence exchanged by a heterologous (and probably more efficient) leader peptide.

The rev-responsiveness of the secreted form ratTHY-lenv was measured by immunoprecipitation of supernatants of human 293T cells cotransfected with a plasmid expressing a secreted form of ratTHY-lenv and the RRE sequence in cis (rTHY-lenvPI-rre) and either CDM7 or pCMVrev. The rTHY-lenvPI-RRE construct was made by PCR using the oligonucleotides cgcggggctagcgcaaagagtaataagtttaac as forward and cgcggatcccttgtattttgtactaata a as reverse primers and the synthetic rTHY-lenv construct as template. After digestion with Nhe1 and Not1 the PCR fragment was cloned into a plasmid containing CD5 leader and RRE sequences. Supernatants of ³⁵S labelled cells were harvested 72 hours post transfection, precipitated with a mouse monoclonal antibody OX7 against rTHY-1 and anti mouse IgG sepharose, and run on a 12% reducing SDS-PAGE.

In this experiment the induction of rTHY-1env by rev was much more prominent and clearcut than in the above-described experiment and strongly suggests that rev is able to translationally regulate transcripts that are suppressed by low-usage codons.

### Rev-independent expression of a rTHY-lenv:immunoglobulin fusion protein

To test whether low-usage codons must be present throughout the whole coding sequence or whether a short region is sufficient to confer rev-responsiveness, a rTHY-lenv:immunoglobulin fusion protein was generated. In this construct the rTHY-lenv gene (without the sequence motif responsible for phosphatidylinositol glycan anchorage) is linked to the human IgG1 hinge, CH2 and CH3 domains. This construct was generated by anchor PCR using primers with Nhe1 and BamHI restriction sites and rTHY-lenv as template. The PCR fragment was cloned into a plasmid containing the leader sequence of the CD5 surface molecule and the hinge, CH2 and CH3 parts of human IgG1 immunoglobulin. A Hind3/Eagl fragment containing the rTHY-lenveg1 insert was subsequently cloned into a pCDM7-derived plasmid with the RRE sequence.

To measure the response of the rTHY-lenv/immunoglobin fusion gene (rTHY-lenveglrre) to rev human 293T cells cotransfected with rTHY-lenveglrre and either pCDM7 or pCMVrev. The rTHY-lenveglrre construct was made by anchor PCR using forward and reverse primers with Nhe1 and BamH1 restriction sites respectively. The PCR fragment was cloned into a plasmid containing a CD5 leader and human IgG1 hinge, CH2 and CH3 domains. Supernatants of ³⁵S labelled cells were harvested 72 hours post transfection, precipitated with a mouse monoclonal antibody OX7 against rTHY-1 and anti mouse IgG sepharose, and run on a 12% reducing SDS-PAGE. The procedures used are described in greater detail below.

As with the product of the rTHY-lenvPI- gene, this rTHY-lenv/immunoglobulin fusion protein is secreted into the supernatant. Thus, this gene should be responsive to rev-induction. However, in contrast to rTHY-lenvPI-, cotransfection of rev in trans induced no or only a negligible increase of rTHY-1enveg1 expression.

The expression of rTHY-1:immunoglobulin fusion protein with native rTHY-1 or HIV envelope codons was measured by immunoprecipitation. Briefly, human 293T cells transfected with either rTHY-lenveg1 (env codons) or rTHY-1wteg1 (native codons). The rTHY-1wteg1 construct was generated in manner similar to that used for the rTHY-lenvegl construct, with the exception that a plasmid containing the native rTHY-1 gene was used as template. Supernatants of ³⁵S labelled cells were harvested 72 hours post transfection, precipitated with a mouse monoclonal antibody OX7 against rTHY-1 and anti mouse IgG sepharose, and run on a 12% reducing SDS-PAGE. THe procedures used in this experiment are described in greater detail below.

Expression levels of rTHY-lenvegl were decreased in comparison to a similar construct with wildtype rTHY-1 as the fusion partner, but were still considerably higher than rTHY-lenv. Accordingly, both parts of the fusion protein influenced expression levels. The addition of rTHY-lenv did not restrict expression to an equal level as seen for rTHY-lenv alone. Thus, regulation by rev appears to be ineffective if protein expression is not almost completely suppressed.

### Codon preference in HIV-1 envelope genes

Direct comparison between codon usage frequency of HIV envelope and highly expressed human genes reveals a striking difference for all twenty amino acids. One simple measure of the statistical significance of this codon preference is the finding that among the nine amino acids with two fold codon degeneracy, the favored third residue is A or U in all nine. The probability that all nine of two equiprobable choices will be the same is approximately 0.004, and hence by any conventional measure the third residue choice cannot be considered random. Further evidence of a skewed codon preference is found among the more degenerate codons, where a strong selection for triplets bearing adenine can be seen. This contrasts with the pattern for highly expressed genes, which favor codons bearing C, or less commonly G, in the third position of codons with three or more fold degeneracy.

The systematic exchange of native codons with codons of highly expressed human genes dramatically increased expression of gp120. A quantitative analysis by ELISA showed that expression of the synthetic gene was at least 25 fold higher in comparison to native gp120 after transient transfection into human 293 cells. The concentration levels in the ELISA experiment shown were rather low. Since an ELISA was used for quantification which is based on gp120 binding to CD4, only native, non-denatured material was detected. This may explain the apparent low expression. Measurement of cytoplasmic mRNA levels demonstrated that the difference in protein expression is due to translational differences and not mRNA stability.

Retroviruses in general do not show a similar preference towards A and T as found for HIV. But if this family was divided into two subgroups, lentiviruses and non-lentiviral retroviruses, a similar preference to A and, less frequently, T, was detected at the third codon position for lentiviruses. Thus, the availing evidence suggests that lentiviruses retain a characteristic pattern of envelope codons not because of an inherent advantage to the reverse transcription or replication of such residues, but rather for some reason peculiar to the physiology of that class of viruses. The major difference between lentiviruses and non-complex retroviruses are additional regulatory and non-essentially accessory genes in lentiviruses, as already mentioned. Thus, one simple explanation for the restriction of envelope expression might be that an important regulatory mechanism of one of these additional molecules is based on it. In fact, it is known that one of these proteins, rev, which most likely has homologues in all lentiviruses. Thus codon usage in viral mRNA is used to create a class of transcripts which is susceptible to the stimulatory action of rev. This hypothesis was proved using a similar strategy as above, but this time codon usage was changed into the inverse direction. Codon usage of a highly expressed cellular gene was substituted with the most frequently used codons in the HIV envelope. As assumed, expression levels were considerably lower in comparison to the native molecule, almost two orders of magnitude when analyzed by immunofluorescence of the surface expressed molecule (see 4.7). If rev was coexpressed in trans and a RRE element was present in cis only a slight induction was found for the surface molecule. However, if THY-1 was expressed as a secreted molecule, the induction by rev was much more prominent, supporting the above hypothesis. This can probably be explained by accumulation of secreted protein in the supernatant, which considerably amplifies the rev effect. If rev only induces a minor increase for surface molecules in general, induction of HIV envelope by rev cannot have the purpose of an increased surface abundance, but rather of an increased intracellular gp160 level. It is completely unclear at the moment why this should be the case.

To test whether small subtotal elements of a gene are sufficient to restrict expression and render it rev-dependent rTHY1env:immunoglobulin fusion proteins were generated, in which only about one third of the total gene had the envelope codon usage. Expression levels of this construct were on an intermediate level, indicating that the rTHY-lenv negative sequence element is not dominant over the immunoglobulin part. This fusion protein was not or only slightly rev-responsive, indicating that only genes almost completely suppressed can be rev-responsive.

Another characteristic feature that was found in the codon frequency tables is a striking underrepresentation of CpG triplets. In a comparative study of codon usage in E. coli, yeast, drosophila and primates it was shown that in a high number of analyzed primate genes the 8 least used codons contain all codons with the CpG dinucleotide sequence. Avoidance of codons containing this dinucleotide motif was also found in the sequence of other retroviruses. It seems plausible that the reason for underrepresentation of CpG-bearing triplets has something to do with avoidance of gene silencing by methylation of CpG cytosines. The expected number of CpG dinucleotides for HIV as a whole is about one fifth that expected on the basis of the base composition. This might indicate that the possibility of high expression is restored, and that the gene in fact has to be highly expressed at some point during viral pathogenesis.

The results presented herein clearly indicate that codon preference has a severe effect on protein levels, and suggest that translational elongation is controlling mammalian gene expression. However, other factors may play ar role. First, abundance of not maximally loaded mRNA's in eukaryotic cells indicates that initiation is rate limiting for translation in at least some cases, since otherwise all transcripts would be completely covered by ribosomes. Furthermore, if ribosome stalling and subsequent mRNA degradation were the mechanism, suppression by rare codons could most likely not be reversed by any regulatory mechanism like the one presented herein. One possible explanation for the influence of both initiation and elongation on translational activity is that the rate of initiation, or access to ribosomes, is controlled in part by cues distributed throughout the RNA, such that the lentiviral codons predispose the RNA to accumulate in a pool of poorly initiated RNAs. However, this limitation need not be kinetic; for example, the choice of codons could influence the probability that a given translation product, once initiated, is properly completed. Under this mechanism, abundance of less favored codons would incur a significant cumulative probability of failure to complete the nascent polypeptide chain. The sequestered RNA would then be lent an improved rate of initiation by the action of rev. Since adenine residues are abundant in rev-responsive transcripts, it could be that RNA adenine methylation mediates this translational suppression.

### Detailed Procedures

The following procedures were used in the above-described experiments.

### Sequence Analysis

Sequence analyses employed the software developed by the University of Wisconsin Computer Group.

### Plasmid constructions

Plasmid constructions employed the following methods. Vectors and insert DNA was digested at a concentration of 0.5 *µ*g/10 *µ*l in the appropriate restriction buffer for 1 - 4 hours (total reaction volume approximately 30 *µ*l). Digested vector was treated with 10% (v/v) of 1 *µ*g/ml calf intestine alkaline phosphatase for 30 min prior to gel electrophoresis. Both vector and insert digests (5 to 10 *µ*l each) were run on a 1.5% low melting agarose gel with TAE buffer. Gel slices containing bands of interest were transferred into a 1.5 ml reaction tube, melted at 65°C and directly added to the ligation without removal of the agarose. Ligations were typically done in a total volume of 25 µl in 1x Low Buffer 1x Ligation Additions with 200-400 U of ligase, 1 µl of vector, and 4 µl of insert. When necessary, 5' overhanging ends were filled by adding 1/10 volume of 250 µM dNTPs and 2-5 U of Klenow polymerase to heat inactivated or phenol extracted digests and incubating for approximately 20 min at room temperature. When necessary, 3' overhanging ends were filled by adding 1/10 volume of 2.5 mM dNTPs and 5-10 U of T4 DNA polymerase to heat inactivated or phenol extracted digests, followed by incubation at 37°C for 30 min. The following buffers were used in these reactions: 10x Low buffer (60 mM Tris HCl, pH 7.5, 60 mM MgCl₂, 50 mM NaCl, 4 mg/ml BSA, 70 mM β-mercaptoethanol, 0.02% NaN₃); 10x Medium buffer (60 mM Tris HCl, pH 7.5, 60 mM MgCl₂, 50 mM NaCl, 4 mg/ml BSA, 70 mM β-mercaptoethanol, 0.02% NaN₃); 10x High buffer (60 mM Tris HCl, pH 7.5, 60 mM MgCl₂, 50 mM NaCl, 4 mg/ml BSA, 70 mM β-mercaptoethanol, 0.02% NaN₃); 10x Ligation additions (1 mM ATP, 20 mM DTT, 1 mg/ml BSA, 10 mM spermidine); 50x TAE (2 M Tris acetate, 50 mM EDTA). Oligonucleotide synthesis and purification

Oligonucleotides were produced on a Milligen 8750 synthesizer (Millipore). The columns were eluted with 1 ml of 30% ammonium hydroxide, and the eluted oligonucleotides were deblocked at 55°C for 6 to 12 hours. After deblockiong, 150 *µ*l of oligonucleotide were precipitated with 10x volume of unsaturated n-butanol in 1.5 ml reaction tubes, followed by centrifugation at 15,000 rpm in a microfuge. The pellet was washed with 70% ethanol and resuspended in 50 *µ*l of H₂O. The concentration was determined by measuring the optical density at 260 nm in a dilution of 1:333 (1 OD₂₆₀ = 30 *µ*g/ml) .

The following oligonucleotides were used for construction of the synthetic gp120 gene (all sequences shown in this text are in 5' to 3' direction).

The following oligonucleotides were used for the construction of the ratTHY-lenv gene.

### Polymerase Chain Reaction

Short, overlapping 15 to 25 mer oligonucleotides annealing at both ends were used to amplify the long oligonuclotides by polymerase chain reaction (PCR). Typical PCR conditions were: 35 cycles, 55°C annealing temperature, 0.2 sec extension time. PCR products were gel purified, phenol extracted, and used in a subsequent PCR to generate longer fragments consisting of two adjacent small fragments. These longer fragments were cloned into a CDM7-derived plasmid containing a leader sequence of the CD5 surface molecule followed by a Nhe1/Pst1/Mlul/EcoR1/BamH1 polylinker.

The following solutions were used in these reactions: 10x PCR buffer (500 mM KC1, 100 mM Tris HC1, pH 7.5, 8 mM MgCl₂, 2 mM each dNTP). The final buffer was complemented with 10% DMSO to increase fidelity of the Taq polymerase.

### Small scale DNA preparation

Transformed bacteria were grown in 3 ml LB cultures for more than 6 hours or overnight. Approximately 1.5 ml of each culture was poured into 1.5 ml microfuge tubes, spun for 20 seconds to pellet cells and resuspended in 200 µl of solution I. Subsequently 400 µl of solution II and 300 µl of solution III were added. The microfuge tubes were capped, mixed and spun for > 30 sec. Supernatants were transferred into fresh tubes and phenol extracted once. DNA was precipitated by filling the tubes with isopropanol, mixing, and spinning in a microfuge for > 2 min. The pellets were rinsed in 70 % ethanol and resuspended in 50 µl dH20 containing 10 µl of RNAse A. The following media and solutions were used in these procedures: LB medium (1.0 % NaCl, 0.5% yeast extract, 1.0% trypton); solution I (10 mM EDTA pH 8.0); solution II (0.2 M NaOH, 1.0% SDS); solution III (2.5 M KOAc, 2.5 M glacial aceatic acid); phenol (pH adjusted to 6.0, overlaid with TE); TE (10 mM Tris HCl, pH 7.5, 1 mM EDTA pH 8.0).

### Large scale DNA Preparation

One liter cultures of transformed bacteria were grown 24 to 36 hours (MC1061p3 transformed with pCDM derivatives) or 12 to 16 hours (MC1061 transformed with pUC derivatives) at 37°C in either M9 bacterial medium (pCDM derivatives) or LB (pUC derivatives). Bacteria were spun down in 1 liter bottles using a Beckman J6 centrifuge at 4,200 rpm for 20 min. The pellet was resuspended in 40 ml of solution I. Subsequently, 80 ml of solution II and 40 ml of solution III were added and the bottles were shaken semivigorously until lumps of 2 to 3 mm size developed. The bottle was spun at 4,200 rpm for 5 min and the supernatant was poured through cheesecloth into a 250 ml bottle. Isopropanol was added to the top and the bottle was spun at 4,200 rpm for 10 min. The pellet was resuspended in 4.1 ml of solution I and added to 4.5 g of cesium chloride, 0.3 ml of 10 mg/ml ethidium bromide, and 0.1 ml of 1% Triton X100 solution. The tubes were spun in a Beckman J2 high speed centrifuge at 10,000 rpm for 5 min. The supernatant was transferred into Beckman Quick Seal ultracentrifuge tubes, which were then sealed and spun in a Beckman ultracentrifuge using a NVT90 fixed angle rotor at 80,000 rpm for > 2.5 hours. The band was extracted by visible light using a 1 ml syringe and 20 gauge needle. An equal volume of dH₂O was added to the extracted material. DNA was extracted once with n-butanol saturated with 1 M sodium chloride, followed by addition of an equal volume of 10 M ammonium acetate/ 1 mM EDTA. The material was poured into a 13 ml snap tube which was tehn filled to the top with absolute ethanol, mixed, and spun in a Beckman J2 centrifuge at 10,000 rpm for 10 min. The pellet was rinsed with 70% ethanol and resuspended in 0.5 to 1 ml of H₂O. The DNA concentration was determined by measuring the optical density at 260 nm in a dilution of 1:200 (1 OD₂₆₀ = 50 *µ*g/ml).

The following media and buffers were used in these procedures: M9 bacterial medium (10 g M9 salts, 10 g casamino acids (hydrolysed), 10 ml M9 additions, 7.5 *µ*g/ml tetracycline (500 *µ*l of a 15 mg/ml stock solution), 12.5 *µ*g/ml ampicillin (125 *µ*l of a 10 mg/ml stock solution); M9 additions (10 mM CaCl₂, 100 mM MgSO₄, 200 *µ*g/ml thiamine, 70% glycerol); LB medium (1.0 % NaCl, 0.5 % yeast extract, 1.0 % trypton); Solution I (10 mM EDTA pH 8.0); Solution II (0.2 M NaOH 1.0 % SDS); Solution III (2.5 M KOAc 2.5 M HOAc)

### Sequencing

Synthetic genes were sequenced by the Sanger dideoxynucleotide method. In brief, 20 to 50 *µ*g doublestranded plasmid DNA were denatured in 0.5 M NaOH for 5 min. Subsequently the DNA was precipitated with 1/10 volume of sodium acetate (pH 5.2) and 2 volumes of ethanol and centrifuged for 5 min. The pellet was washed with 70% ethanol and resuspended at a concentration of 1 *µ*g/*µ*l. The annealing reaction was carried out with 4 *µ*g of template DNA and 40 ng of primer in 1x annealing buffer in a final volume of 10 *µ*l. The reaction was heated to 65°C and slowly cooled to 37°C. In a separate tube 1 µl of 0.1 M DTT, 2 µl of labeling mix, 0.75 *µ*l of dH²0, 1 µl of [³⁵S] dATP (10 uCi), and 0.25 *µ*l of Sequenase^{™} (12 U/*µ*l) were added for each reaction. Five *µ*l of this six were added to each annealed primer-template tube and incubated for 5 min at room temperature. For each labeling reaction 2.5 µl of each of the 4 termination mixes were added on a Terasaki plate and prewarmed at 37°C. At the end of the incubation period 3.5 *µ*l of labeling reaction were added to each of the 4 termination mixes. After 5 min, 4 µl of stop solution were added to each reaction and the Terasaki plate was incubated at 80°C for 10 min in an oven. The sequencing reactions were run on 5% denaturing polyacrylamide gel. An acrylamide solution was prepared by adding 200 ml of 10x TBE buffer and 957 ml of dH₂0 to 100 g of acrylamide:bisacrylamide (29:1). 5% polyacrylamide 46% urea and 1x TBE gel was prepared by combining 38 ml of acrylamide solution and 28 g urea. Polymerization was initiated by the addition of 400 *µ*l of 10% ammonium peroxodisulfate and 60 µl of TEMED. Gels were poured using silanized glass plates and sharktooth combs and run in 1x TBE buffer at 60 to 100 W for 2 to 4 hours (depending on the region to be read). Gels were transferred to Whatman blotting paper, dried at 80°C for about 1 hour, and exposed to x-ray film at room temperature. Typically exposure time was 12 hours. The following solutions were used in these procedures: 5x Annealing buffer (200 mM Tris HCl, pH 7.5, 100 mM MgCl₂, 250 mM NaCl); Labelling Mix (7.5 *µ*M each dCTP, dGTP,and dTTP); Termination Mixes (80 *µ*M each dNTP, 50 mM NaCl, 8 *µ*M ddNTP (one each)); Stop solution (95% formamide, 20 mM EDTA, 0.05 % bromphenol blue, 0.05 % xylencyanol); 5x TBE (0.9 M Tris borate, 20 mM EDTA); Polyacrylamide solution (96.7 g polyacrylamide, 3.3 g bisacrylamide, 200 ml 1x TBE, 957 ml dH₂O).

### RNA isolation

Cytoplasmic RNA was isolated from calcium phosphate transfected 293T cells 36 hours post transfection and from vaccinia infected Hela cells 16 hours post infection essentially as described by Gilman. (Gilman Preparation of cytoplasmic RNA from tissue culture cells. In Current Protocols in Molecular Biology, Ausubel et al, eds., Wiley & Sons, New York, 1992). Briefly, cells were lysed in 400 µl lysis buffer, nuclei were spun out, and SDS and proteinase K were added to 0.2% and 0.2 mg/ml respectively. The cytoplasmic extracts were incubated at 37°C for 20 min, phenol/chloroform extracted twice, and precipitated. The RNA was dissolved in 100 µl buffer I and incubated at 37°C for 20 min. The reaction was stopped by adding 25 µl stop buffer and precipitated again.

The following solutions were used in this procedure: Lysis Buffer (TE containing with 50 mM Tris pH 8.0, 100 mM NaCl, 5 mM MgCl₂, 0.5% NP40); Buffer I (TE buffer with 10 mM MgCl₂, 1 mM DTT, 0.5 U/µl placental RNAse inhibitor, 0.1 U/µl RNAse free DNAse I); Stop buffer (50 mM EDTA 1.5 M NaOAc 1.0 % SDS).

### Slot blot analysis

For slot blot analysis 10 µg of cytoplasmic RNA was dissolved in 50 µl dH₂O to which 150 µl of 10x SSC/18% formaldehyde were added. The solubilized RNA was then incubated at 65°C for 15 min and spotted onto with a slot blot apparatus. Radioactively labelled probes of 1.5 kb gp120IIIb and syngp120mn fragments were used for hybridization. Each of the two fragments was random labelled in a 50 µl reaction with 10 µl of 5x oligo-labelling buffer, 8 µl of 2.5 mg/ml BSA, 4 µl of α[³²P]-dCTP (20 uCi/µl; 6000 Ci/mmol), and 5 U of Klenow fragment. After 1 to 3 hours incubation at 37°C 100 µl of TE were added and unincorporated α[³²P]-dCTP was eliminated using G50 spin column. Activity was measured in a Beckman beta-counter, and equal specific activities were used for hybridization. Membranes were pre-hybridized for 2 hours and hybridized for 12 to 24 hours at 42°C with 0.5 x 10⁶ cpm probe per ml hybridization fluid. The membrane was washed twice (5 min) with washing buffer I at room temperature, for one hour in washing buffer II at 65°C, and then exposed to x-ray film. Similar results were obtained using a 1.1 kb Notl/Sfil fragment of pCDM7 containing the 3 untranslated region. Control hybridizations were done in parallel with a random-labelled human beta-actin probe. RNA expression was quantitated by scanning the hybridized nitrocellulose membranes with a Magnetic Dynamics phosphorimager.

The following solutions were used in this procedure:
5x Oligo-labelling buffer (250 mM Tris HCl, pH 8.0, 25 mM MgCl₂, 5 mM β-mercaptoethanol, 2 mM dATP, 2mM dGTP, 2mM dTTP, 1 M Hepes pH 6.6, 1 mg/ml hexanucleotides [dNTP]6); Hybridization Solution (_M sodium phosphate, 250 mM NaCl, 7% SDS, 1 mM EDTA, 5% dextrane sulfate, 50% formamide, 100 µg/ml denatured salmon sperm DNA); Washing buffer I (2x SSC,
0.1% SDS); Washing buffer II (0.5x SSC, 0.1 % SDS); 20x SSC (3 M NaCl, 0.3 M Na₃citrate, pH adjusted to 7.0).

### Vaccinia recombination

Vaccinia recombination used a modification of the of the method described by Romeo and Seed (Romeo and Seed, Cell, 64: 1037, 1991). Briefly, CV1 cells at 70 to 90% confluency were infected with 1 to 3 µl of a wildtype vaccinia stock WR (2 x 10⁸ pfu/ml) for 1 hour in culture medium without calf serum. After 24 hours, the cells were transfected by calcium phosphate with 25 µg TKG plasmid DNA per dish. After an additional 24 to 48 hours the cells were scraped off the plate, spun down, and resuspended in a volume of 1 ml. After 3 freeze/thaw cycles trypsin was added to 0.05 mg/ml and lysates were incubated for 20 min. A dilution series of 10, 1 and 0.1 *µ*l of this lysate was used to infect small dishes (6 cm) of CV1 cells, that had been pretreated with 12.5 *µ*g/ml mycophenolic acid, 0.25 mg/ml xanthin and 1.36 mg/ml hypoxanthine for 6 hours. Infected cells were cultured for 2 to 3 days, and subsequently stained with the monoclonal antibody NEA9301 against gp120 and an alkaline phosphatase conjugated secondary antibody. Cells were incubated with 0.33 mg/ml NBT and 0.16 mg/ml BCIP in AP-buffer and finally overlaid with 1% agarose in PBS. Positive plaques were picked and resuspended in 100 µl Tris pH 9.0. The plaque purification was repeated once. To produce high titer stocks the infection was slowly scaled up. Finally, one large plate of Hela cells was infected with half of the virus of the previous round. Infected cells were detached in 3 ml of PBS, lysed with a Dounce homogenizer and cleared from larger debris by centrifugation. VPE-8 recombinant vaccinia stocks were kindly provided by the AIDS repository, Rockville, MD, and express HIV-1 IIIB gp120 under the 7.5 mixed early/late promoter (Earl et al., J. Virol., 65:31, 1991). In all experiments with recombinant vaccina cells were infected at a multiplicity of infection of at least 10.

The following solution was used in this procedure: AP buffer (100 mM Tris HCl, pH 9.5, 100 mM NaCl, 5 mM MgCl₂)

### Cell culture

The monkey kidney carcinoma cell lines CV1 and Cos7, the human kidney carcinoma cell line 293T, and the human cervix carcinoma cell line Hela were obtained from the American Tissue Typing Collection and were maintained in supplemented IMDM. They were kept on 10 cm tissue culture plates and typically split 1:5 to 1:20 every 3 to 4 days. The following medium was used in this procedure:
Supplemented IMDM (90% Iscove's modified Dulbecco Medium, 10% calf serum, iron-complemented, heat inactivated 30 min 56°C, 0.3 mg/ml L-glutamine, 25 µg/ml gentamycin 0.5 mM β-mercaptoethanol (pH adjusted with 5 M NaOH, 0.5 ml)).

### Transfection

Calcium phosphate transfection of 293T cells was performed by slowly adding and under vortexing 10 *µ*g plasmid DNA in 250 *µ*l 0.25 M CaCl₂ to the same volume of 2x HEBS buffer while vortexing. After incubation for 10 to 30 min at room temperature the DNA precipitate was added to a small dish of 50 to 70% confluent cells. In cotransfection experiments with rev, cells were transfected with 10 *µ*g gp120IIIb, gp120IIIbrre, syngp120mnrre or rTHY-lenveglrre and 10 *µ*g of pCMVrev or CDM7 plasmid DNA.

The following solutions were used in this procedure: 2x HEBS buffer (280 mM NaCl, 10 mM KCl, 1.5 mM sterile filtered); 0.25 mM CaCl₂ (autoclaved).

### Immunoprecipitation

After 48 to 60 hours medium was exchanged and cells were incubated for additional 12 hours in Cys/Met-free medium containing 200 µCi of ³⁵S-translabel. Supernatants were harvested and spun for 15 min at 3000 rpm to remove debris. After addition of protease inhibitors leupeptin, aprotinin and PMSF to 2.5 *µ*g/ml, 50 *µ*g/ml, 100 *µ*g/ml respectively, 1 ml of supernatant was incubated with either 10 *µ*l of packed protein A sepharose alone (rTHY-lenveglrre) or with protein A sepharose and 3 *µ*g of a purified CD4/immunoglobulin fusion protein (kindly provided by Behring) (all gp120 constructs) at 4°C for 12 hours on a rotator. Subsequently the protein A beads were washed 5 times for 5 to 15 min each time. After the final wash 10 µl of loading buffer containing was added, samples were boiled for 3 min and applied on 7% (all gp120 constructs) or 10% (rTHY-lenveglrre) SDS polyacrylamide gels (TRIS pH 8.8 buffer in the resolving, TRIS pH 6.8 buffer in the stacking gel, TRIS-glycin running buffer, Maniatis et al. 1989). Gels were fixed in 10% acetic acid and 10 % methanol, incubated with Amplify for 20 min, dried and exposed for 12 hours.

The following buffers and solutions were used in this procedure: Wash buffer (100 mM Tris, pH 7.5, 150 mM NaCl, 5 mM CaCl₂, 1% NP-40); 5x Running Buffer (125 mM Tris, 1.25 M Glycin, 0.5% SDS); Loading buffer (10 % glycerol, 4% SDS, 4% β-mercaptoethanol, 0.02 % bromphenol blue).

### Immunofluorescence

293T cells were transfected by calcium phosphate coprecipitation and analyzed for surface THY-1 expression after 3 days. After detachment with 1 mM EDTA/PBS, cells were stained with the monoclonal antibody OX-7 in a dilution of 1:250 at 4°C for 20 min, washed with PBS and subsequently incubated with a 1:500 dilution of a FITC-conjugated goat anti-mouse immunoglobulin antiserum. Cells were washed again, resuspended in 0.5 ml of a fixing solution, and analyzed on a EPICS XL cytofluorometer (Coulter).

The following solutions were used in this procedure:
PBS (137 mM NaCl, 2.7 mM KCl, 4.3 mM Na₂HPO₄, 1.4 mM KH₂PO₄, pH adjusted to 7.4); Fixing solution (2% formaldehyde in PBS).

### ELISA

The concentration of gp120 in culture supernatants was determined using CD4-coated ELISA plates and goat anti-gp120 antisera in the soluble phase. Supernatants of 293T cells transfected by calcium phosphate were harvested after 4 days, spun at 3000 rpm for 10 min to remove debris and incubated for 12 hours at 4°C on the plates. After 6 washes with PBS 100 µl of goat anti-gp120 antisera diluted 1:200 were added for 2 hours. The plates were washed again and incubated for 2 hours with a peroxidase-conjugated rabbit anti-goat IgG antiserum 1:1000. Subsequently the plates were washed and incubated for 30 min with 100 µl of substrate solution containing 2 mg/ml o-phenylenediamine in sodium citrate buffer. The reaction was finally stopped with 100 µl of 4 M sulfuric acid. Plates were read at 490 nm with a Coulter microplate reader. Purified recombinant gp12oIIIb was used as a control. The following buffers and solutions were used in this procedure: Wash buffer (0.1% NP40 in PBS); Substrate solution (2 mg/ml o-phenylenediamine in sodium citrate buffer).

## Claims

1. A method for preparing a synthetic gene encoding a protein normally expressed in mammalian cells,
wherein at least 50% of the non-preferred codons and less preferred codons present in the natural gene encoding said protein have been replaced by preferred codons, encoding the same amino acids,
said preferred codons being selected from the group consisting of gcc, cgc, aac, gac, tgc, cag, ggc, cac, atc, ctg, aag, ccc, ttc, age, acc, tac and gtg, said less preferred codons being selected from the group consisting of ggg, att, ctc, tcc and gtc, and said non-preferred codons being all codons other than said preferred codons and said less preferred codons,
wherein the synthetic gene is capable of expressing said mammalian protein at a level which is at least 110% of that expressed by the natural gene in *an in vitro* mammalian cell culture system under identical conditions.

2. A synthetic gene obtainable by a method according to claim 1, wherein said protein is an HIV protein.

3. A synthetic gene of claim 2 wherein said protein is selected from the group consisting of gag, pol, and env.

4. A synthetic gene of claim 2, wherein said protein is gp120 or gp160.

## Patentansprüche

1. Verfahren zur Herstellung eines für ein normalerweise in Säugerzellen exprimiertes Protein codierenden synthetischen Gens,
wobei wenigstens 50% der im für das Protein codierenden natürlichen Gen vorhandenen nicht bevorzugten Codons und weniger bevorzugten Codons gegen die gleichen Aminosäuren codierende bevorzugte Codons ausgetauscht sind,
wobei die bevorzugten Codons ausgewählt sind aus der Gruppe bestehend aus gcc, cgc, aac, gac, tgc, cag, ggc, cac, atc, ctg, aag, ccc, ttc, agc, acc, tac und gtg, die weniger bevorzugten Codons ausgewählt sind aus der Gruppe bestehend aus ggg, att, ctc, tcc und gtc und es sich bei den nicht bevorzugten Codons um alle von den bevorzugten Codons und den weniger bevorzugten Codons verschiedenen Codons handelt,
wobei das synthetische Gen dazu in der Lage ist, das Säugerprotein auf einem Niveau zu exprimieren, das mindestens 110% des Expressionsniveaus des natürlichen Gens in einem Säugerzellenkultursystem *in vitro* unter identischen Bedingungen entspricht.

2. Synthetisches Gen, erhältlich mit einem Verfahren nach Anspruch 1, wobei es sich bei dem Protein um ein HIV-Protein handelt.

3. Synthetisches Gen nach Anspruch 2, wobei das Protein ausgewählt ist aus der Gruppe bestehend aus gag, pol und env.

4. Synthetisches Gen nach Anspruch 2, wobei es sich bei dem Protein um gp120 oder gp160 handelt.

## Revendications

1. Procédé de préparation d'un gène synthétique codant pour une protéine normalement exprimée dans des cellules de mammifère,
dans lequel au moins 50 % des codons non préférés et des codons moins préférés présents dans le gène naturel codant pour ladite protéine ont été remplacés par les codons préférés, codant pour les mêmes acides aminés,
lesdits codons préférés étant choisis dans le groupe constitué de gcc, cgc, aac, gac, tgc, cag, ggc, cac, ate, ctg, aag, ccc, ttc, age, ace, tac et gtg, lesdits codons moins préférés étant choisis dans le groupe constitué de ggg, art, etc, tec et gtc, et lesdits codons non préférés étant tous les codons autres que lesdits codons préférés et lesdits codons moins préférés,
dans lequel le gène synthétique est susceptible d'exprimer ladite protéine mammalienne à un niveau qui est au moins 110 % de celui exprimé par le gène naturel dans un système de culture de cellules de mammifère *in vitro* dans des conditions identiques.

2. Gène synthétique pouvant être obtenu par un procédé selon la revendication 1, dans lequel ladite protéine est une protéine HTV.

3. Gène synthétique selon la revendication 2, dans lequel ladite protéine est choisie dans le groupe constitué de gag, pol et env.

4. Gène synthétique selon la revendication 2, dans lequel ladite protéine est gpl20 ou gpl 60.
